(19)

**Europäisches Patentamt**
**European Patent Office**
**Office européen des brevets**

(11) **EP 3 923 215 A1**

(12) **EUROPEAN PATENT APPLICATION**

(43) Date of publication:
**15.12.2021 Bulletin 2021/50**

(51) Int Cl.:
**G06Q 10/06** *(2012.01)*

(21) Application number: **20397508.1**

(22) Date of filing: **10.06.2020**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Firstbeat Analytics OY
40100 Jyväskylä (FI)**

(72) Inventors:
• **Seppänen, Mikko
40100 JYVÄSKYLÄ (FI)**
• **Toiviainen, Maunu
40100 JYVÄSKYLÄ (FI)**

(74) Representative: **Berggren Oy
P.O. Box 16
Eteläinen Rautatiekatu 10A
00101 Helsinki (FI)**

(54) **A METHOD, AN APPARATUS AND A COMPUTER PROGRAM PRODUCT FOR PROVIDING A NEXT WORKOUT RECOMMENDATION**

(57) Providing a next workout recommendation (NWR) comprises calculating an activity class for a user; determining a total training load target based on the activity class and a training goal; determining labels for performed workouts, wherein a label describes a training effect of a workout and wherein the labels are further divided into intensity categories of *aerobic low load, aerobic high load and anaerobic load;* detecting a training load distribution (TLD), wherein the TLD comprises a cumulative training load sum for the intensity categories; determining the intensity category based on recovery time from the previous training, a weekly training load compared to a weekly training load target and the TLD, to select a label for the NWR; and determining the NWR based on the labels of the previous workouts, determined weekly training load and the TLD, wherein the NWR comprises at least an aerobic/anaerobic training effect target and/or the selected label.

**FIG. 1**

EP 3 923 215 A1

**Description**

Technical field

The application relates to providing a next workout recommendation, and more particularly, but not exclusively, the application relates to a method, an apparatus and a computer program product for providing a next workout recommendation.

Background

Fitness and wellbeing can be improved by systematic workout routines. A personal trainer or a fitness educated expert can plan and support person's workout routines by planning programs and providing workout recommendations. Automated software solutions exist to perform similar tasks. Even the most thorough trainer or expert cannot detect or notice all the variables related to the current fitness level of the user, nor can they summarize all the training history aspects relevant for the next workout to be as efficient and fruitful as possible.

Summary

It is an aim to provide a workout recommendation according to a set goal adaptively, taking into account recorded workout history and current condition of a user.
According to an aspect of the invention a method for providing a next workout recommendation (NWR) comprises,

- calculating an activity class for a user based on training history data and/or present fitness level of the user;
- determining a total training load target based on the activity class and a training goal; wherein the training goal relates to maintaining or changing the fitness level of the user;
- determining labels for performed workouts from the training history data, wherein a label describes a training effect of a workout, based on one or more of the measured intensities during workouts, calculated aerobic training effect and calculated anaerobic training effect; wherein the labels are further divided into intensity categories of *aerobic low load, aerobic high load and anaerobic load,* wherein each of the intensity categories comprises one or more labels,
- detecting a training load distribution from the training history data, wherein the training load distribution comprises a cumulative training load sum for the intensity categories of *aerobic low load, aerobic high load* and *anaerobic load,*
- calculating a weekly training load target based on the activity class and the training goal,
- calculating a weekly training load based on the training history data,
- determining the intensity category for the NWR based on recovery time from the previous training, a weekly training load compared to the weekly training load target and the training load distribution, and
- determining the NWR based on the labels of the previous workouts, determined weekly training load and the training load distribution, wherein the NWR comprises at least an aerobic training effect target, an anaerobic training effect target and/or the selected label.

According to an aspect of the invention an apparatus comprises means for implementing the method according to the aspects of the invention.
An apparatus for providing a next workout recommendation (NWR) comprises,

- an arrangement configured to calculate an activity class for a user based on training history data and/or present fitness level of the user;
- an arrangement configured to determine a total training load target based on the activity class and a training goal; wherein the training goal relates to maintaining or changing the fitness level of the user;
- an arrangement configured to determine labels for performed workouts from the training history data, wherein a label describes a training effect of a workout, based on one or more of the measured intensities during workouts, calculated aerobic training effect and calculated anaerobic training effect; wherein the labels are further divided into intensity categories of *aerobic low load, aerobic high load and anaerobic load,* wherein each of the intensity categories comprises one or more labels,
- an arrangement configured to detect a training load distribution from the training history data, wherein the training load distribution comprises a cumulative training load sum for the intensity categories of *aerobic low load, aerobic high load* and *anaerobic load,*
- an arrangement configured to calculate a weekly training load target based on the activity class and the training goal,
- an arrangement configured to calculate a weekly training load based on the training history data,

- an arrangement configured to determine the intensity category for the NWR based on recovery time from the previous training, a weekly training load compared to the weekly training load target and the training load distribution, and
- an arrangement configured to determine the NWR based on the labels of the previous workouts, determined weekly training load and the training load distribution, wherein the NWR comprises at least an aerobic training effect target, an anaerobic training effect target and/or the selected label.

According to an aspect of the invention a computer program product comprises means for implementing the method according to the aspects of the invention. A computer program product for providing a next workout recommendation (NWR) comprises executable instructions which, when executed by a processor, cause implementation of:

- calculating an activity class for a user based on training history data and/or present fitness level of the user;
- determining a total training load target based on the activity class and a training goal; wherein the training goal relates to maintaining or changing the fitness level of the user;
- determining labels for performed workouts from the training history data, wherein a label describes a training effect of a workout, based on one or more of the measured intensities during workouts, calculated aerobic training effect and calculated anaerobic training effect; wherein the labels are further divided into intensity categories of *aerobic low load, aerobic high load and anaerobic load,* wherein each of the intensity categories comprises one or more labels,
- detecting a training load distribution from the training history data, wherein the training load distribution comprises a cumulative training load sum for the intensity categories of *aerobic low load, aerobic high load* and *anaerobic load,*
- calculating a weekly training load target based on the activity class and the training goal,
- calculating a weekly training load based on the training history data,
- determining the intensity category for the NWR based on recovery time from the previous training, a weekly training load compared to the weekly training load target and the training load distribution, and
- determining the NWR based on the labels of the previous workouts, determined weekly training load and the training load distribution, wherein the NWR comprises at least an aerobic training effect target, an anaerobic training effect target and/or the selected label.

Brief description of the drawings

In the following embodiments are described in more detail with the accompanying drawings of which:

| | |
|---|---|
| Figure 1 | shows an exemplary flowchart for providing a next workout recommendation. |
| Figure 2 | shows an exemplary flowchart for providing a next workout recommendation. |
| Figure 3A | shows the first part of an exemplary flowchart for providing a next workout recommendation. |
| Figure 3B | shows the second part of the exemplary flowchart for providing a next workout recommendation. |
| Figure 3C | shows the third part of the exemplary flowchart for providing a next workout recommendation. |
| Figure 3D | shows the fourth part of the exemplary flowchart for providing a next workout recommendation. |
| Figure 4 | shows an exemplary anaerobic feedback logic based on a training effect. |
| Figure 5 | shows an exemplary aerobic feedback logic based on a training effect. |
| Figure 6 | represents an example selection logic for determining the aerobic and anaerobic workout label for each activity. |
| Figure 7 | shows exemplary anaerobic feedback logic phrases based on training effects. |
| Figures 8A and 8B | show an exemplary aerobic feedback logic phrases based on training effects. |
| Figure 9 | displays an example user interface showing a training load distribution |
| Figure 10 | shows an exemplary alignment between an activity class and a weekly training load target. |

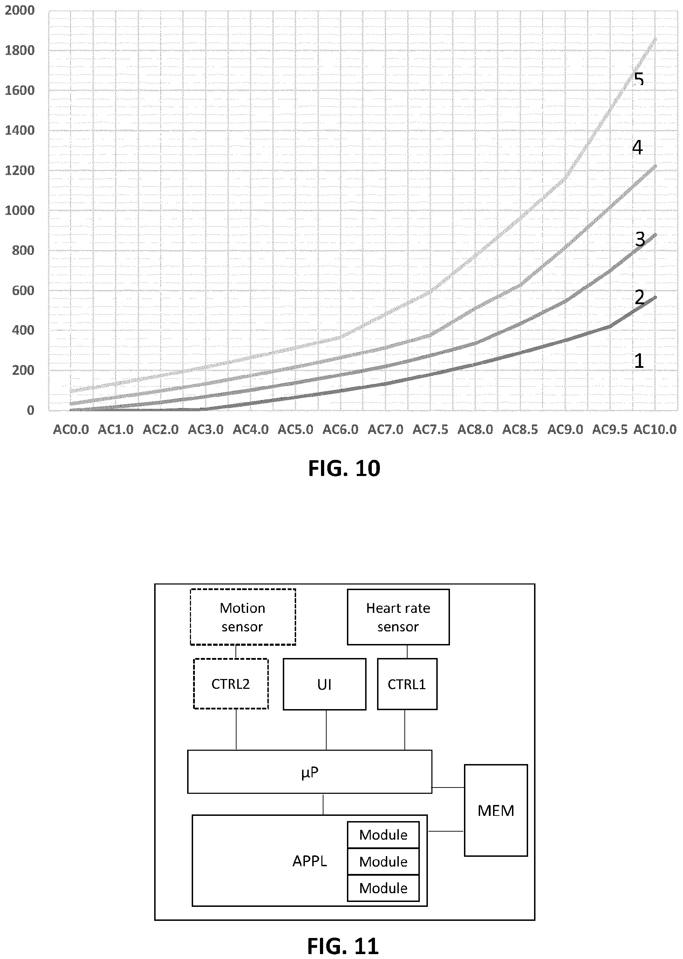

Figure 11                    shows an exemplary apparatus for providing the next workout recommendation according to an embodiment

The Figures, examples and embodiments relating to the above Figures illustrate aspects of the invention or alternatively, are presented for better understanding the claimed invention, their subject-matter, features and related issues.

Detailed description

Next workout recommendation NWR may be used to prescribe safe and effective workouts for a user taking into account the current physical state, the training history, the fitness level and the training goal of the user. Thereafter, the NWR may determine the load target for the next training cycle (e.g. calendar week) based on realized WTL during past few weeks and the overall training history of the user. The target load may be easy, moderate or hard. Next, the training load from previous 4-14 days is compared to the determined target in order to select suitable training load target for today's workout. Based on these calculations also a label and a workout structure for the NWR are determined. The NWR may aim to include weekly variation in the longer term training program and thereby provides changes in the WTL. If the changes are set to zero, a steady weekly target WTL may be obtained.

**A fitness level**

The term fitness level refers to a physical ability or property of a user. The fitness level of a user may be measured from a training history data including information on performed training(s) of the user. Alternatively or in addition, a user specific background information may have effect on the fitness level of the user. Background parameters may include age, gender, height and weight of a user. In addition background parameters include the current date information. The background parameters may be used for determining maximum heart rate maxHR of the user. The background parameters may be used for determining minimum heart rate minHR and/or resting heart rate restHR of the user. Determined background parameters may affect the current fitness level of the user. The fitness level of a user may be measured online during a training or a workout performed by the user. The fitness level may be measured via maximum aerobic capacity of a user, like a maximum oxygen consumption (VO2max) or a maximum metabolic equivalent (maxMET), for example. Running speed proportioned to user's personal lactate threshold speed or speed corresponding to VO2max may be vVO2max. vVO2max may correspond to average speed, for example over a 3 kilometer running race. A cycling power proportioned to a user's personal functional threshold power may be called FTP. VO2max, restHR, heart rate variation HRV recovery state, all-day HRV and/or other training history parameters may be used for determining the current fitness level of the user.

The terms training, exercise and workout may be used equally to describe a workout or contents of it, which is performed or to be performed by a user. A training plan and a training program refer to a training plan/program including two or more next workout recommendations.

**An activity class**

A present activity class of a user may be determined using background information, such as age, gender, height and/or weight; a fitness level of the user; and/or activity/training history data of the user. The activity class describes user's fitness level and training tolerance. The determined activity classes may be classified. For example, a scale of 0-10 may be used, wherein 0 represents a sedentary user and 10 represents highly fit and trained user. Each activity class may have its own specific training load target, which may comprise a range. A training load target may comprise a lowest limit for a training load of the activity class. In addition, a training load target may comprise an upper limit for a training load of the activity class. If training is periodized, for example on a weekly basis using simple easy-medium-hard periodization in terms of load, then each of these weeks have different training load targets even if activity class remained the same.

The history length required for determining activity class may change based on a metric used. However, the history length for calculating chronic training load depicting a user's training tolerance is typically calculated with approximately a 3-10 week time period, preferably a 4-8 week time period.

In addition to the history length, the weight of different days may vary within a selected time window. This is shown, for example, in the way that the most distant days and/or the most closest days may have a lower weight than the days in the middle, between the closest and the most distant days of the selected time window, when calculating average weekly load. By this procedure single days dropping out from the selected time window may have less effect on the activity class. Similarly, this kind of procedure may be used to prevent activity class increasing rapidly in cases where a user has done really hard workouts during the past few days. From a physiological point of view, this kind of procedure may recommend an easy training period rather than increasing the user's load targets, which would be done if activity class increased based on a short time window or higher weights on workouts on the closest days.

In the onboarding phase with a new user and/or where the information from the user's training is still limited - a forecast or prediction may be employed for considering the training tolerance. In this prediction a conservative approach may be applied where the activity class is not allowed to increase too rapidly even though the available information from that rather short period of history shows high training loads. This kind of limiting of activity class is intended to prevent too fast progression of training recommendations considering the load and duration of recommended workouts, for example.

Next workout recommendations (NWRs) may be based on an activity class that is somewhat insensitive to daily fluctuations in calculated fitness levels. The training history of the user may be weighted in order to better represent the user's training tolerance. In an embodiment, there may be an exception, related to an onboarding phase for a new user, where the rate of increase of the activity class may be, even heavily, limited during the first four weeks of the training program.

The following is an exemplary method for determining an activity class. This calculation may be performed by first determining an initial activity class based on either a training load value or a maximal met value, whichever one is higher. Depending on the training history available, the training load value may be either an unscaled monthly training load (meaning, an entire month of training load), or a scaled monthly load.

Selection of these values is made based on the amount of training history data available, where, on day 0, the date of the first workout in training history, activity class based on a maximal met value or unscaled monthly training load. Between days 0-26, the activity class is determined by a linear interpolation between two values, being the greater of: a maximal met and an unscaled training load; and a scaled training load. The scaled training load is based on the scaled monthly training load and the unscaled training load is based on the unscaled monthly training load. On day 27 and beyond, the activity class is set to the highest activity class value between the maximal met and the scaled monthly load.

A scaled monthly training load may be calculated by computing a weighted daily average training load of the user. Older training load data are given different weightings according to tables 1 and 2, depending on how much training history data is available. The weight of the training load may be 1 for recent (0-6 days) and increase weekly as the workout age. For example, a 3-4 week old (21-34 days) training load may have a weight of 4. As the workout further ages, the weight of the training load may decline correspondingly, such that it is again 1 after 49-55 days and 0 if the workout is older than 56 days. If there is less user data available, the weighting may be different, but preserve the same structure of weight change. For example, a user data from 28 days would have training load weight of 1 for 0-6 days, weight of 2 for 7-13 days, weight of 2 for 14-20 days and weight of 1 for 21-27 days in the workout history.

**Example on a calculation of an activity class**

Activity may be calculated for example using the following two variables:

1. ac_by_maxmet_or_unscaled_ml which is the largest of the two values ac_unscaled_ml and ac_by_maxmet, where

    a) ac_unscaled_ml is the activity class calculated based on the unscaled monthly load (i.e., the monthly load is not multiplied by four during the first training week, etc.)
    b) ac_by_maxmet is the activity class based on the maxMET

2. 56d_ac which is the largest of the two values ac_scaled_ml and ac_by_maxmet, where

    a) ac_scaled_ml is the activity class calculated based on the scaled monthly load (see more detailed description later)
    b) ac_by_maxmet is the activity class based on the maxMET

Once these two values have been calculated, activity class is obtained as follows:

- On day 0 (i.e., the date of the first workout in training history), activity class is assigned to the value ac_by_maxmet_or_unscaled_ml .
- On day 27 (i.e., the 28th day of the training history) and afterwards, activity class is set to the value 56d_ac.
- On days 1-26, activity class is obtained via linear interpolation between the two values ac_by_maxmet_or_unscaled_ml and average_ac.

As mentioned above, 56d_ac is the largest of the two values ac_scaled_ml and ac_by_maxmet, where ac_scaled_ml is calculated in a following manner:

1. Compute the daily average training load of the user as a weighted average where each workout is weighted depending on its age, for example as shown in the tables 1 and 2, where exercises on the current day and 6 preceding days get a weight of 1, exercises in the preceding 7 day period get a weight of 2, etc..

Table 1. Average training load weights based on exercise age

| Age of exercise (days) when user has data from 56 or more days | Weight |
|---|---|
| 0-6 | 1 |
| 7-13 | 2 |
| 14-20 | 3 |
| 21-34 | 4 |
| 35-41 | 3 |
| 42-48 | 2 |
| 49-55 | 1 |
| 56- | 0 |

Table 2. Average training load weights based on limited exercise age

| Age of exercise (days) when user has data from 28 days | Weight |
|---|---|
| 0-6 | 1 |
| 7-13 | 2 |
| 14-20 | 2 |
| 21-27 | 1 |

2. Scale the daily average training load to a monthly training load (MTL) Sum. In a situation, where the known training history spans N ≤ 6 days, multiply by 4N, otherwise multiply by 28.

Load based AC determination may be done by comparing ac_unscaled_ml and ac_scaled_ml to the values presented in the following table 3. For example, if the ac_scaled_ml is 1120 units after 28 days of training that would guarantee at least an activity class of 7.5. Of course, if user's VO2max is really high, then AC can be even higher. Units may be ml/kg, when aerobic load is based on EPOC, or different arbitrary units, when aerobic load is based on TRIMP. An activity class may have a monthly training load (MTL) limit. The activity class (AC) may be higher for higher training loads. AC and WTL limits are shown in Figure 10.

Table 3. exemplary monthly training load limits for different activity classes.

| Activity class | MTL-limit |
|---|---|
| AC0.0 | 0 |
| AC1.0 | 17 |
| AC2.0 | 40 |
| AC3.0 | 68 |
| AC4.0 | 400 |
| AC5.0 | 548 |
| AC6.0 | 704 |
| AC7.0 | 876 |
| AC7.5 | 1092 |
| AC8.0 | 1336 |

(continued)

| Activity class | MTL-limit |
|---|---|
| AC8.5 | 1731 |
| AC9.0 | 2177 |
| AC9.5 | 2792 |
| AC10.0 | 3512 |

**A training goal**

The term training goal describes a desired effect for example to a fitness level of a user. The training goal may be to maintain or change the fitness level of a user. The training goal may include rate of change of the fitness level. Training goal may be may be set or selected to be, for example, to maintain, increase or increase fast the fitness level of a user. Training goal may also be for example tapering. Training goal may be set either manually or automatically. For example, in a use case where the user is allowed to set a target time for different running distances, if the target time is much faster than the current (predicted) ability, the program code may be configured to cause selection of "increase fast" training goal instead of "increase" training goal. User may also have a possibility to set the target date for an event. In an exemplary embodiment the user may primarily follow training plan with either "increase" or "increase fast" training goal but the program code may be configured to cause selection of the training goal "tapering" once there is only three weeks preparation time left. Accordingly, training goal may be for example changing the recovery state of the user in addition to the training goal of maintaining/changing the fitness level of the user. A next workout recommendation (NWR) is determined for the user such that it supports the training goal. The training goal may be time dependent. For example, time for achieving a set training goal may be determined. The training goal may comprise short-term and long-term training goals. The term training program refers to multiple following next workout recommendations.

**A training load**

A training load is a measure of how much the body's homeostasis has been disturbed with training, and it describes a physiological exercise load to a user by a given workout. A training load target may be determined. The training load target level may be dependent on a present activity class, which relates to a fitness level of a user. Absolute training load targets may be higher for higher activity class values than for lower activity class values. A weekly training load (WTL) may be determined by summing the highest training load (training load peak) achieved in each workout session performed during a week. A week may refer to a training cycle of 4-14 days, for example. Although a target weekly training load (WTL) is referred to in this application, it may refer to a target training load for n-days, where n is integer, for example n = 4-14, thus any number of days between 4-6 or 8-12 instead of seven days (a week). Correspondingly, a target monthly training load (MTL) may refer to a training target load for number of days, e.g. 24-38 days. Further, the weekly/monthly training load(s), or corresponding n-day training loads, may be used as a parameter(s) for determinations and calculations. WTL or a training load target of a block or of a month may be upgraded, being higher compared to user's prior training(s), in order to improve and increase the present fitness level or activity class of the user. For higher activity class values or fitness level of a user, the training load target may not increase linearly, but less or modest, in order to avoid recommendation of too heavy exercise.

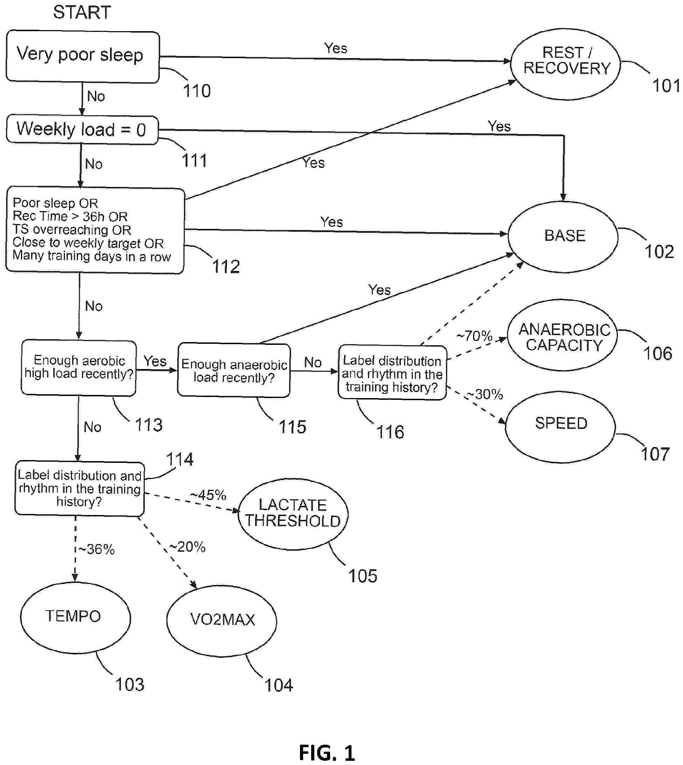

Figures 1-2, 3A-D illustrate flowcharts for implementation of embodiments, which are disclosed herein. It is noted that the order of phases illustrated in Figures is not required, but the various phases may be performed out of the illustrated order. In addition, certain phases may be skipped, different phases may be added or substituted, or selected phases or group of phases may be performed in a separate application, following the embodiments described herein.

The determined NWR may be based on an activity class AC, a training history, a training load target, and a training goal of the user. The activity class AC describes user's fitness level and may take into account user's training history. The determined activity class may be classified or scaled as 1-10. Each activity class may have its own specific training load target. The training load target may comprise limit values. Training goal may be set by the user and it may relate to maintaining a fitness level, or changing a fitness level of a user. In addition, the training goal may relate to changing a recovery state of the user. The training goal may be set for the fitness level to *increase, maintain* or *increase fast*.

**A training history**

Training history data comprise data on previous exercises in a chronological order, as performed by the use.

The training history data comprises exercise data from a number of past days. The training history data may comprise date and time information, a training load peak information, workout induced recovery time and fitness level for each performed training, as well as type of the training. The training load peak information, the resource recovery time and the fitness level may be calculated in real time by a real time calculation engine that comprises as an input an inter-beat interval and/or HR level data, and optionally an external workload, like speed, changes in altitude or external power output. Type of exercise may comprise identifying the type of training. The date and time information may comprise the time of starting the training, the time of ending the training and/or times of training periods. The training load peak information, the resource recovery time and the fitness level may be calculated in real time by a real time calculation engine. Input for the real time calculation engine may comprise an inter-beat interval and/or heart rate level data. In addition the input may comprise an external workload, like speed, altitude or external power output. An activity class of a user may be updated based on the training history data. A training goal may be inputted or determined based on inputted target time or other parameters, for example.

Training history data may be used for determining maximum metabolic equivalent maxMET and/or maximum oxygen consumption VO2max of the user. These may have effect on the current fitness level of the user. An activity class of a user may be updated based on the training history data. A training goal may be inputted or determined based on inputted target time or other parameters, for example.

**A recovery state**

A recovery state describes how well a user has physiologically recovered from the recent workout(s). A recovery state may be measured with an analysis of heart rate variation HRV, such as during sleep, or during a discrete recovery test measurement, and it may be individually scaled by taking into account the typical values and range of HRV for each user. Therefore, a recovery state may be presented relative to a user's typical values, or as a trend. The trend may include information on whether the recovery state is improving or getting worse. The amount of a recent training load may contribute to a determination of a recovery state, and be presented in a form of a number of hours it will take for a user to fully recover from the most recent workout.

**A training status**

To be able to make decisions on future trainings/workouts a user needs to know the current trajectory of their training, referred to as the training status (TS). Training status is determined based on three main parameters: a recovery state, a fitness level and a training load. The training load may relate to a current short-term training load, and a change in short-term training load with respect to a previous training.

**Training readiness**

As described above, training status may tell a user about the trajectory of their past training. Overreaching status is a clear case which may be taken into account in next workout recommendation; I.e. in a case of "overreaching" rest or recovery workouts may be recommended until a better recovered status is reached. These may comprise at least "recovery " and "productive " statuses. On the other hand - for example, reaching productive status in the history does not always guarantee a good readiness to perform in the coming workouts. This may be the case for example when a user has done a hard workout on a previous day meaning that his or her recovery processes are not yet complete. Similar case may be a situation where the past training has been productive, but a user has slept poorly during previous night. In both of these examples previous training may show nice progress, but still the instantaneous training readiness may be poor. Accordingly, in one exemplary embodiment NWR may utilize information on the measured instantaneous training readiness. Training readiness may be determined using information of one or more of the following: recovery time from previous exercise, sleep quality and/or sleep duration during last night, current training load level, sleep duration and/or sleep quality during the previous nights, stability of sleeping rhythm, measured or estimated stress levels during previous days, measured heart rate variability during previous day or days, or user's self-reported energy level or mood. Estimated high training readiness can be used to trigger harder than normal workouts or training days. For example, whereas the default training rhythm may require two successive recovery or base workouts in a row before a high aerobic session may be prescribed, High training readiness state may form an exception where only one recovery or base workout is required in between two high aerobic sessions. Vice versa, information on poor training readiness may be used to trigger easier training. For example, if estimated training readiness is "moderate " then only base or recovery workout prescriptions (or rest) may be allowed. Similarly, if estimated training readiness is poor then only recovery prescriptions (or rest) may be allowed.

**An injury risk**

Injury risk reflects a user's capability to exercise based on their past training history. Taking into account a user's recent week's training load compared to their training tolerance in addition to their recovery state allows for determination of an overall risk of injury from training excessively. An example method of calculating injury risk is disclosed in applicant's own publication US20190214144. In this example, injury risk is determined on a 0-100 scale, where 0 represents a very high injury risk. Therefore, similar to training status or sleep score, if the risk of injury is high, such as a score within the range of 0-30, then only rest or recovery workouts may be recommended. It should be obvious to one skilled in the art that other criteria or modifications to the next workout recommendation based on injury risk scores may be made based on what is considered an acceptable risk of injury relative to the intensity of the next workout.

**An aerobic training effect**

An aerobic training effect (aerTE) is a scaled value that describes the physiological impact or effect the training has on aerobic performance. The aerTE may be calculated by analyzing the aerobic intensity of the exercise, for example by using a cumulative training load measure, like an excess post-exercise oxygen consumption (EPOC) or a training impulse (TRIMP), and scaling the training load sum based on commonly known quantities of aerobic work in different exercises. Aerobic training loads may be calculated from successive aerobic training effects.

**An anaerobic training effect**

An anaerobic training effect (anTE) is a scaled value that describes the physiological impact or effect the training has on anaerobic performance. The anTE may be calculated by analyzing the anaerobic sum, determined by identifying and analyzing periods of high-intensity intervals, and by scaling that based on commonly known quantities of anaerobic work in different exercises. Anaerobic training loads may be calculated from successive anaerobic training effects.

**Scaling of training effects (aerTE and anTE)**

An anaerobic training effect may be determined by comparing the anaerobic sum with an anaerobic work scale. The anaerobic sum may relate to a cumulative sum of a work done during workouts performed by a user. An aerobic training effect may be determined by comparing the aerobic sum with an aerobic work scale. A main training effect may be determined as being the higher of the aerobic- and anaerobic training effect values. A ratio between the anaerobic training effect and the aerobic training effect may be determined. The ratio between the anTE and the aerTE may represent the proportional benefit of a workout on energy production pathways, for example. A physical fitness level of a user may be taken into account when evaluating the *anaerobic load* of the performed exercise. In principle, a user with higher fitness level (or activity level) needs to get higher anaerobic sum to achieve similar training effect. In similar fashion, the performed aerobic sum is scaled during exercise by comparing measured aerobic sum to reference values for aerobic work.

The calculated training effects aerTE and anTE can be expressed, for example, as scaled values 0-5, where 0 denotes no exercise and 5 denotes a very heavy exercise.

A full description of a method for calculating a training effect values is presented in applicant's own patent publication US2017143262A1. This publication, as well as other publications referred to in this description, are included herein.

**Labeling logic**

Labels, or workout labels, may be determined based on measured intensity during exercise and optionally the training effect accumulated during exercise. The measured intensity may be instantaneously measured in real-time during a workout. The intensity may comprise a single intensity parameter; or a combination of heart rate and one or more intensity parameters. The intensity parameters may be: heart rate proportioned either to the user's personal lactate threshold heart rate or to maximal heart rate; running speed proportioned either to the user's personal lactate threshold (LT) speed or to speed corresponding to VO2max; cycling power proportioned either to the user's personal functional threshold power (FTP) or to maximal aerobic power (for example the user's highest 5 minute average power). Applicant has also developed so called "modified intensity" (see US2017143262A1), which enables tracking intensities even above 100% of VO2max just based on a heart rate information. Modified intensity may also be used in labeling of workouts.

The workout labels can be, for example, the following: Recovery, Base (endurance), Tempo, Lactate Threshold, VO2max, Anaerobic Capacity and Speed. All of these terms are well-known terms with generally well-known definitions. Low intensity workouts with a short duration may be labeled as Recovery. Low intensity workouts with extended duration typically enhance fat usage in the body as well as muscular endurance and thus maybe labeled us Base. Workouts with a vigorous intensity not yet close to Lactate Threshold improve for example cardiorespiratory capacity and muscular endurance especially if they have extended duration and may be labeled as Tempo workouts. Even though the benefits of Tempo workouts may have many similarities with Lactate Threshold workouts they have slightly lower intensity and thus allow a user to prolong the workout considerably which enables development of muscular endurance needed in prolonged distances (including for example marathon) as well as enables developing movement efficiency and economy at the marathon specific pace. Workouts with slightly higher effort when compared to tempo workouts may be labeled as Lactate Threshold workouts as they directly challenge body's capability to fight against lactic acid accumulation during workouts. Workouts with an intensity about like that threshold typically accumulate muscular/blood lactic acid to levels significantly above the Lactate Threshold. This type of intensity maximizes the usage of oxygen in the body as well as develops users ability to work efficiently under increasing lactic acid levels in the body. Workouts at this type of intensity (above lactate threshold but below or equal to the workload at VO2max is achieved) may be labeled as VO2max workouts.

Workout intensities above VO2max develop anaerobic capacity. Workouts having intensity above VO2max but still significantly below users (estimated) maximal speed can thus be labeled as anaerobic capacity. Workout intensities close to or equal to user's maximal speed also develop his or her ability for example to run or ride faster and maybe thus labeled as speed workouts.

Labels may be used to define and describe primary and secondary benefits of the exercise in order to provide feedback to a user about the exact physiological benefits of a single exercise. The primary and secondary benefits may be called the primary and secondary labels correspondingly. In addition, labels may be used to analyze a training load distribution. Labels are more accurate than "time in zone analysis" in determining the true benefits of the exercise as labels isolate and differentiate different phases and structures from the exercise.

Labeling of workouts may be implemented in such a way that it enables both accurate verbal descriptions of the formed workouts as well as summarizing the benefit into a single label. Accordingly there may be even tens of feedbacks even though a number of labels is limited to 7, for example. Workout labels may be based on aerobic and anaerobic feedback phrases - each feedback phrase may have a corresponding workout label. Each workout may accumulate both *aerobic* and *anaerobic load.* A determined aerobic training load may be transferred to a selected aerobic label. Similarly a determined anaerobic training load may be transferred to a selected anaerobic label. Each workout may get two labels, one to represent aerobic training load and one to represent anaerobic training load. Based on the cumulative training load sum for both anaerobic and aerobic training load, and the identified workout label, the respective training load units are transferred to the particular anaerobic and aerobic label(s). Over multiple workouts, training load units can accumulate within specific labels and identify the proportion of the types of training over a given period.

In an exemplary embodiment, a method may be used to provide additional feedback on the specific benefit of each workout. The results of the workout may be supplemented by additional feedback beyond showing a singular training effect value. The additional feedback, which may be referred to as a "training label", may be determined using the steps shown in Figures 4-6. Figure 4 shows an exemplary anaerobic feedback logic based on a training effect, Figure 5 shows an exemplary aerobic feedback logic based on a training effect, and Figure 6 represents an example selection logic for determining the aerobic and anaerobic workout label for each activity with three label groups, feedback phrase numbers and workout labels. A first part of the process, as described previously, is to determine a numerical value of training effect, for both an aerobic training effect and anaerobic training effect.

In an exemplary embodiment, a determination of feedback phrase is made separately for both the aerobic training and anaerobic training effect.

**Aerobic Feedback**

Aerobic feedback phrases may be determined based on the aerobic training effect value and information on the intensity distribution of exercise. In running, intensity distribution may be analyzed in terms of heart rate and optionally running speed. In cycling, intensity distribution may be analyzed in terms of heart rate and optionally cycling power.

Analysis of intensity distribution may be based on various factors related to the intensity a user exercises at and the length of time spent in certain intensity zones (in terms of, for example, heart rate, running speed, or cycling power), or the duration of the period of intensity relative to the total duration of the activity.

Intensity may be measured as a relative intensity, benchmarked against lactate threshold heart rate (LTHR) or lactate threshold speed (LT speed) or Functional Threshold Power (FTP) of the user. These measures may be manually input by the user or automatically calculated based on earlier workouts.

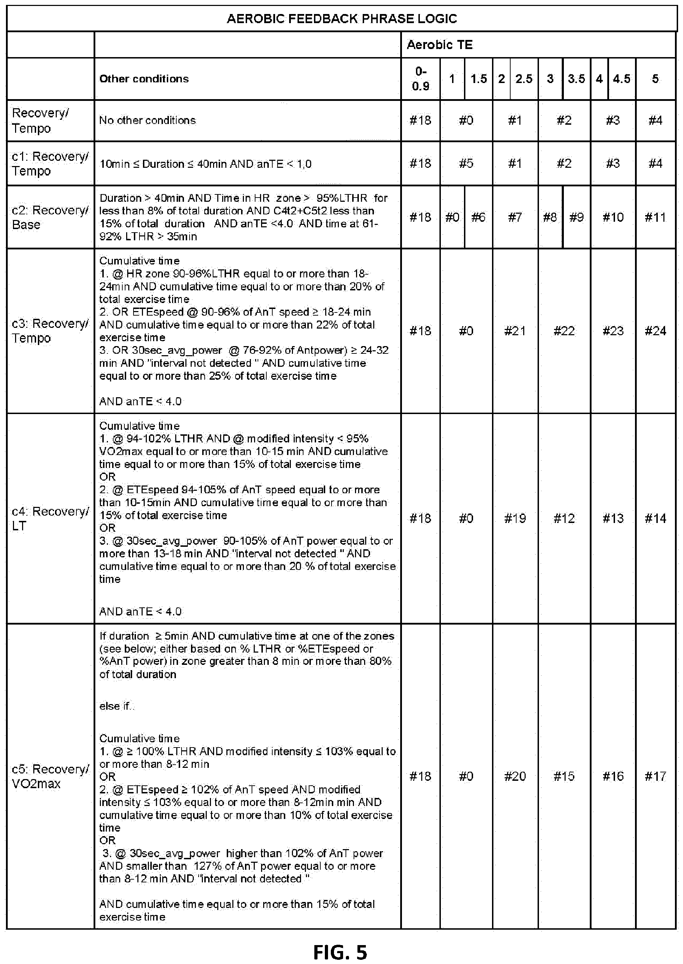

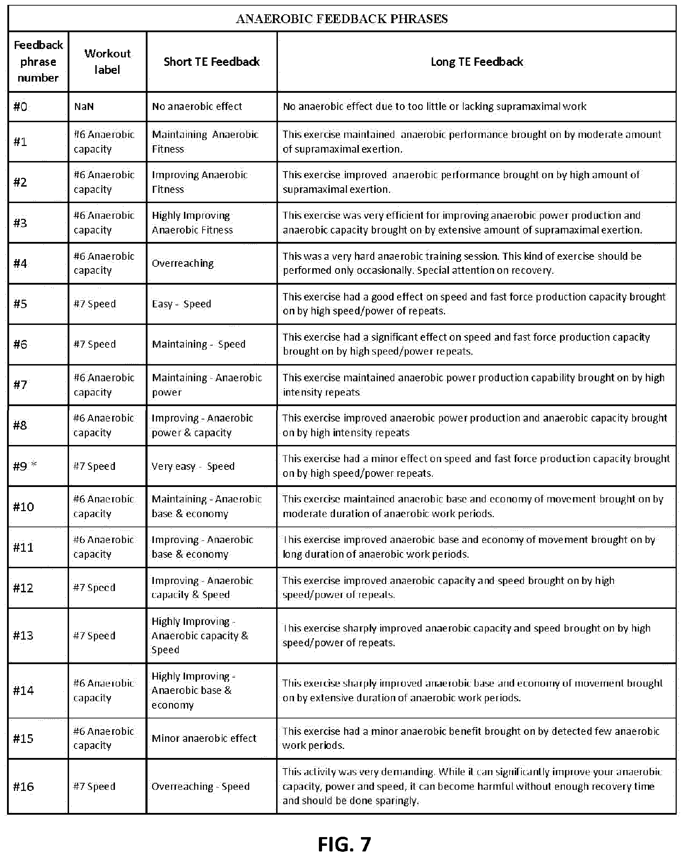

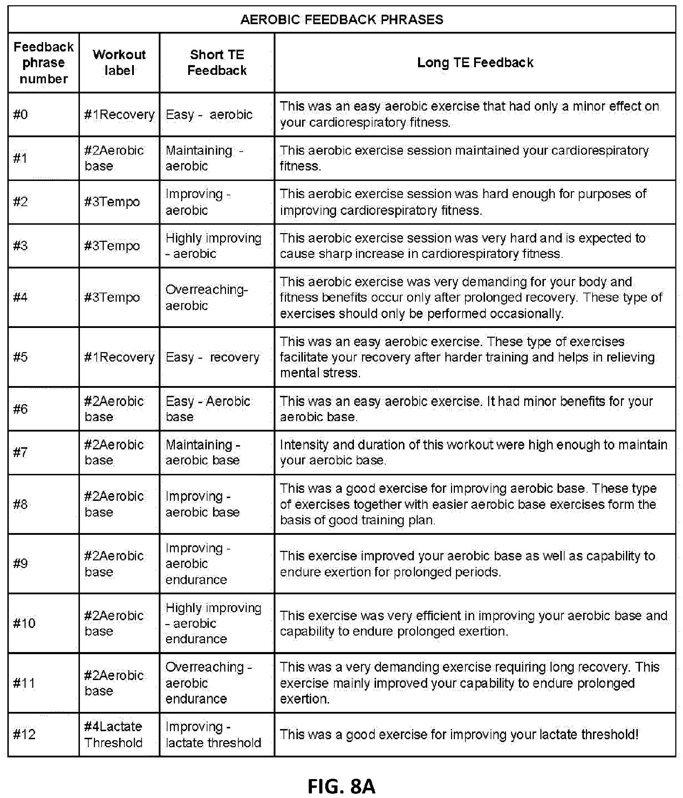

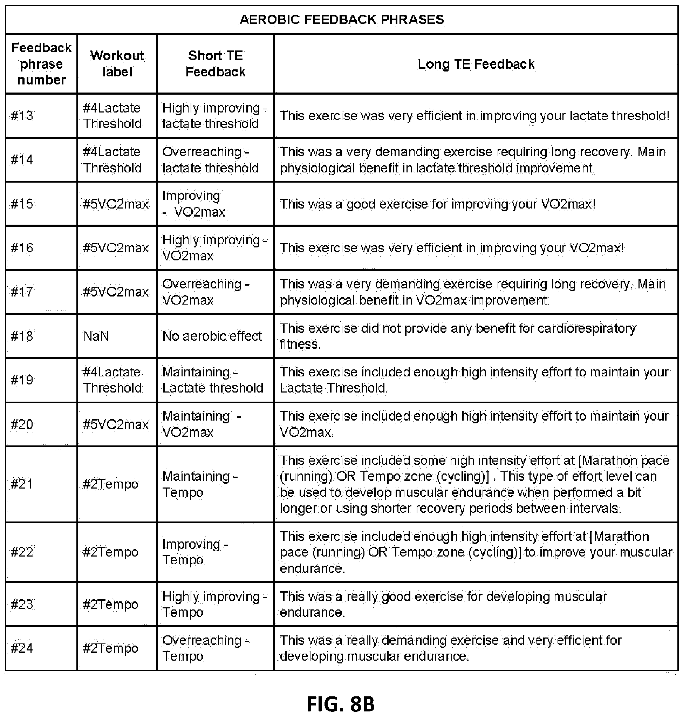

In an alternative embodiment, the results of the workout may be supplemented by additional feedback beyond showing a training effect. An exemplary embodiment comprises showing a numerical value of training effect value for an aerobic training effect, and for an anaerobic training effect. Further, the exemplary embodiment comprises determining and showing feedback phrases related to both aerobic and anaerobic work to the user. Aerobic Feedback phrases telling the aerobic benefit of a workout is demonstrated in Figures 7-8. Figure 7 shows the exemplary anaerobic feedback logic phrases based on a training effect. Figures 8A and 8B show an exemplary aerobic feedback logic phrases based on a training effect. Aerobic feedback phrases may be determined initially based on the aerobic training effect value. Additionally, the aerobic feedback phrases may be chosen based on an extended criteria. The extended criteria may be based on relative intensity wherein the intensity may be benchmarked against lactate threshold heart rate (LTHR) or lactate threshold speed (LT speed) or Functional Threshold Power (FTP) of the user. Examples of the extended criteria are shown in Figure 5. In cases where these values are not known they may be estimated using, for example, the following formulas

$$FTP \text{ power (W)} = ((maxMET*3.5*weight-350)/12.24)*0.828$$

$$LT \text{ speed (m/s)} = (maxMET*3.5-3.5)/12*0.828 + 0.1486$$

If LTHR not known, use default 90%Hrmax as LTHR,
where maxMET may be determined based on a user's fitness level, or VO2max. The factors and values of the factors in the above example formulas are based on scientifically reported values, which may be improved with data based optimization.
Some of the criteria may also be based on the corresponding anaerobic training effect value.

**Exemplary embodiments based on tables 4 and 5**

According to an example embodiment, a cyclist, having an activity class of AC8, performs a workout consisting of a variable intensity warm-up, 2 x 20m in repeats at an intensity close to or above his lactate threshold (FTP) intensity and a short cool-down. During a warm-up his intensity is mostly lowish staying mostly below 90%LTHR and below 76% FTP. During the latter part of the warm-up the user's intensity increases above 76% FTP but that does not yet raise aerobic TE to 2.0. Since time at recovery and base training zones (=61-92% HRmax) does not accumulate above the 35 min threshold, and short recovery does not accumulate much time at Tempo zone or higher zones either, aerobic feedback phrase changes from #18 (no aerobic effect) into #5 (easy recovery). At that time point, no anaerobic training effect has accumulated and thus anaerobic feedback phrase stays at #0 (no anaerobic effect). Thus, the primary label (benefit) after the warm-up is #1 - "Recovery". After a few minutes of first FTP-power repeat, while the aerobic TE reached the level of 2.0, an aerobic feedback phrase turns into #1 (maintaining aerobic) which triggers also the change of the primary label into "Aerobic base", since the exercise has still accumulated very little of high intensity effort when compared to total working time (35 min) so far. Also the anaerobic TE is still 0.0 at that point. However, about 5 minutes later, when repeat has taken approximately 8 min aerobic training effect (aerTE) reaches 3.0 level and accumulated time at 61-92% HRmax -intensity is less than 35 min. An aerobic feedback phrase #8 is thus not possible and an aerobic feedback phrase turns into #2 ("Improving Aerobic"), which in turn changes the primary label from "Base" into "Tempo", since the anaerobic training effect (anTE) is still 0.0. At the end part of first 20 min repeat the cyclist has accumulated enough time (13 min 20 sec) considering his activity class at lactate threshold HR zone (94-102% LTHR) without modified intensity simultaneously reaching too high values (>95%). Accordingly, the aerobic feedback phrase changes into #12 - " improving lactate threshold", which also causes the primary label to turn into #4 - "Lactate Threshold". After a short recovery period the user starts their second repeat where the intensity is slightly higher when compared to the first one. During the second repeat aerobic feedback phrase turns into #13 -" highly improving lactate threshold", but it does not cause changes in the primary label. Due to the increased effort time at VO2max, a training zone starts to accumulate. Also the anaerobic training effect is starting to increase. At the very end of the second repeat, the user reaches a trigger limit which is 10 min 30 sec at VO2max HR zone. Accordingly, the aerobic feedback phrase turn into #16 - "highly improving VO2max". In the comparison of the aerobic training effect (4.5) and the anaerobic training effect (3.5), aerobic energy systems are regarded as the main beneficiary. Accordingly, the primary label (primary benefit) at the end of workout is #5 (VO2max) - not anaerobic capacity even though the anaerobic work of workout has focused on that ability. All aerobic load of that exercise (208 units) is allocated to "VO2max" -label and *aerobic high load* category. All anaerobic load (116 units) is allocated to *anaerobic load* intensity category, since the workout exceeded anTE 1.0 level. The *anaerobic load* is further allocated under "anaerobic capacity" label, since a detected supramaximal effort did not reach high enough level in order to be regarded as a speed training (Anaerobic feedback phrase = #2).

**Anaerobic feedback**

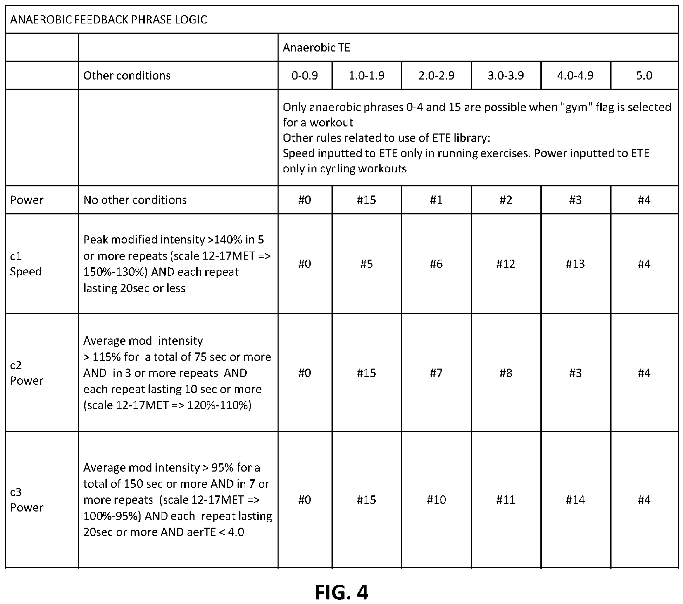

Anaerobic feedback phrases illustrate the anaerobic benefit of a workout. Examples of the anaerobic feedback are shown in Figures 4, 6, and 7. The anaerobic feedback phrases may be determined using the determined anaerobic training effect, in addition to criteria related to modified intensity measurements as well as quantity, duration, and intensity of detected anaerobic intervals. This may also include external workload intensity, such as based on speed from running workouts or power input from cycling workouts.

**Summary feedback**

Based on both the aerobic and anaerobic feedback phrases determined for each workout, a summary of the current workout may be determined by means of labels. The purpose of the labels is to summarize the benefits of the workout with respect to the physiological systems developed. Aerobic workout labels 1-5, comprise for example, recovery training, aerobic base training, tempo training, lactate threshold training and VO2max training. Anaerobic workout labels 6-7, comprise for example anaerobic capacity training or speed training.

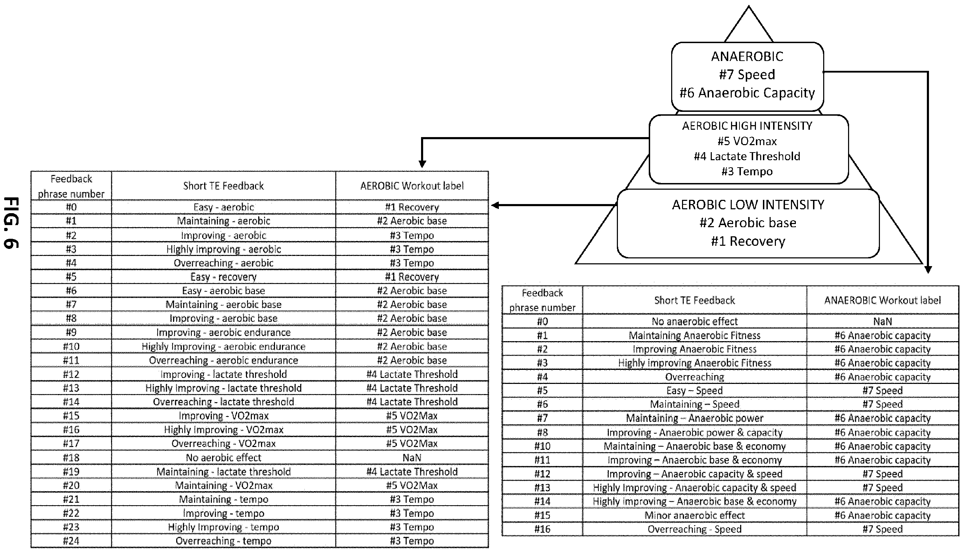

Summarizing the current training workout by determining the benefits as load for one or more labels of said training workout may be described, as illustrated in Figure 6, for aerobic energy production (labels 1-5) comprising, for example, recovery training, aerobic base training, tempo training, LT training and VO2max training; or for anaerobic energy production (labels 6-7) comprising, for example, anaerobic capacity training and speed training.

In an exemplary embodiment presented in Figure 6 each workout may get two labels (meaning two main benefits): 1) one of the *aerobic low* or *aerobic high* intensities comprising labels 1-5; and 2) one of the *anaerobic* intensities comprising labels 6-7. For example, an easy jogging workout including ten 50m sprints may get label 2 ("Base") from the aerobic labels and label 7 "Sprint" from the anaerobic labels.

**The primary label (primary benefit) of workout**

From a coaching point of view it is often useful to also point out the primary label (being primary benefit) of each workout. The selection of the primary label of the workout may be performed based on the calculated aerobic and anaerobic training effect using for example the following criteria:

- When anaerobic TE (anTE) $\geq$ 3.0, select an anaerobic label (label 6 or 7) as the primary label if it is greater or equal to aerobic TE (aerTE) - 0.5.
- If anTE < 3.0, then the primary label is whichever is higher, aerobic label (label 1, 2, 3, 4 or 5) or anTE.
- If anTE = aerTE then the anaerobic effect is always selected as the primary label.
- If the anaerobic label is Speed and anTE $\geq$ 2.0, select anaerobic label as primary label, if it is greater or equal to aerTE - 1 (being aerTE minus one).
- If the anaerobic label is Speed and anTE < 2.0, then the workout label is based on whichever training effect is higher, aerTE or anTE.
- In "strength training mode", select the anaerobic label as workout label if AnTE $\geq$ 1, if it is greater or equal to aerTE - 1.5. (being aer TE minus 1.5)

"Strength training" is an example of a mode, which may be selected whenever user selects a sport mode for an exercise that is characterized by lifting weights or where user has to use high amount of force in short bouts.

There may optionally be presented a "secondary" label corresponding to a training effect label that is not selected as the primary label, but in addition to the primary label.

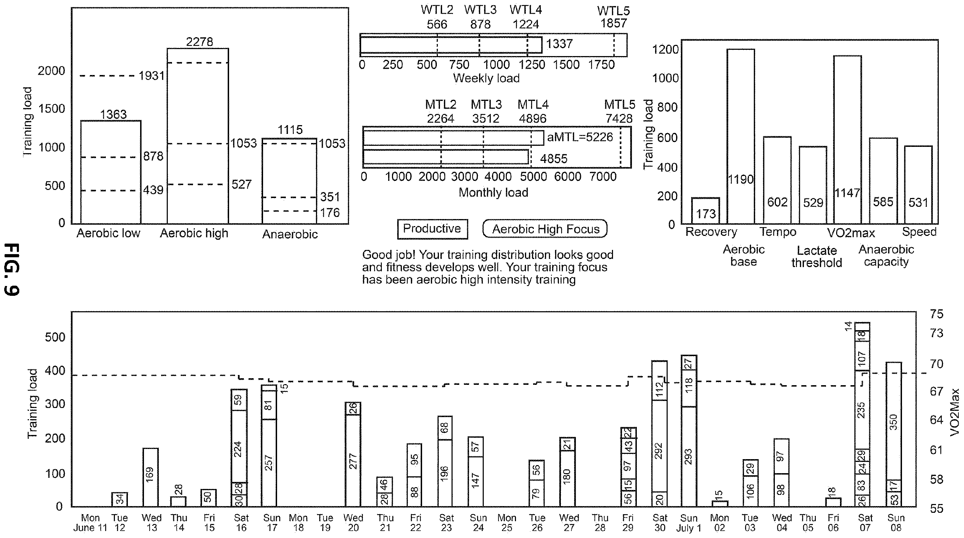

To illustrate the balance of a user's training, the training history may be summarized. In an exemplary embodiment, a summarized training history may comprise summing all of the training load accumulated and distributed to the different training labels. The training load is distributed unweighted, regardless of whether it has the "primary" or "secondary" training load. For example a workout with aerTE of 3.3 and anTE of 1.1 may get a primary label from some of the aerobic labels 1-5. Regardless of that, the secondary anaerobic effect #6 or #7 may also be taken into account in the training load distribution. Any duration of historical training load may be presented to the user. Figure 9 shows an example of a cumulative training load feedback. As shown in Figure 9, historical training load may be shown in a daily calendar, a weekly (7-day) illustration, a monthly (28-day) illustration, or a custom date as specified by the user. The training load may be illustrated in a yearly (365-day) distribution. The training load distribution may be shown per each label, or alternatively by diving labels into groups, for example into 3 intensity categories (as shown in Figure 6): labels 1-2 into *aerobic low load* category, labels 3-5 into *aerobic high load* category and labels 6-7 into *anaerobic load* category. A training load distribution is described in more detail in the following.

The purpose of calculating the training load distribution based on intensity categories is to track whether a user is sufficiently stressing different body systems or stressing them in a balanced manner.

In a specific situation, where a feedback has phrase #0, with the workload label of NaN as shown on Figure 7, the workout load may not be taken into account in the training load distribution.

**A training load distribution**

The training load distribution may be divided into intensity categories. Training load may be divided into three intensity categories (*aerobic low load, aerobic high load* and *anaerobic load*) based on the workout label of each exercise. The training load distribution may be called an intensity category distribution. The *aerobic low load* may generally be defined as low-intensity aerobic training, for example, aerobic exercise at a heart rate below 80% of a user's maximum heart rate. This kind of training forms the basis of any endurance training plan as this type of training allows high training volumes. The aerobic high workout may be considered as a workout that involves a higher heart rate than the defined intensity threshold of the *aerobic low load* workout, but does not belong in the category of being an anaerobic workout, anaerobic workout being identified, for example, as described above and in Figure 6. The aerobic high training intensities may be used to optimize aerobic capacity. However, regardless of being efficient in optimizing aerobic (cardiorespiratory) capacity this kind of training increase training load rapidly and can thus not be repeated as often as the aerobic base workouts. Accordingly, the aerobic low intensity training creates base fitness that allows training on a daily basis (or even several daily workouts) in the long term, which is why this type of training forms the basis of endurance training. An anaerobic training is performed at intensities beyond a user's VO2max. They are needed to optimize performance as this kind of training improves, for example, exercise economy, as well as capability to (repeated) sprints which are crucial characteristics in endurance sports. Accordingly all training intensities are relevant when it comes to development and optimization of endurance performance.

**A monthly training load (MTL) target**

Activity class (AC) based target values for monthly training load (MTL) may be determined for each category. As a general rule of thumb: training is in good balance when the training load in each category is within their target limits. Here, categories refer to the previously mentioned intensity categories.

During an early phase of the training when the user has not yet trained for a full month (28 days) or there is not enough training data history, the target values shown below in table 4 are multiplied with L/28, where L < 28 is the number of days from the oldest recorded exercise to the current date.

Table 4: Exemplary monthly training load (MTL) target values

| TARGET VALUES | Aerobic Low | Aerobic High | Anaerobic |
|---|---|---|---|
| **Minimal limit** | 12.5% of MTL 3.0 | 15% of MTL 3.0 | 5% of MTL 3.0* |
| **Target lower limit** | 25% of MTL 3.0 | 30% of MTL 3.0 | 10% of MTL 3.0* |
| **Target upper limit** | 55% of MTL 3.0 | 60% of MTL 3.0 | 30% of MTL 3.0 |
| **Very high limit** | 80% of MTL 3.0 | 80% of MTL 3.0 | 60% of MTL 3.0 |
| MTL limits are determined based on monthly determined activity class (AC).<br>* IF AC $\leq$ 7 minimal limit, and target lower limit for anaerobic is 0. | | | |

**Training load distribution feedback**

Based on the actual accumulated training load and its distribution, exemplary feedback sentences may be provided according to the rules described in the below tables 5 and 6. In addition to the feedback itself, training load distribution feedbacks may be used for determination of the next workout recommendation (NWR). For example, if an *aerobic high shortage state,* meaning a shortage compared to the target value, is detected in the *aerobic high load,* this information may be used as an input for the next workout recommendation. This may cause *aerobic high* workouts getting more weight or being prescribed more frequently for the following NWRs.

There are some potential exceptions to the rules shown below:

- Below targets: Monthly training load is under 65% of MTL 3.0 (this value may be scaled during the onboarding phase like other training load target values).

- "Approaching targets" is provided instead of below targets if WTL $\geq$ 2.5.

- Above targets: 145% or higher of MTL 3.0 (this value is intentionally not scaled during the onboarding phase).

- Focus is selected for that category which is proportionally closest to "upper-limit" or "very high limit".

- Additional exceptions would be to not allow 1) "Above targets", nor 2) "Approaching targets", if the below table suggests "#2 Aer. low Shortage" (I.e. Then show "#2 Aer. low Shortage" to a user).

Table 5

| # | Distribution feedback | Example of long feedback |
|---|---|---|
| 0 | No result | |
| 1 | Below Targets | "Your long term training load is below optimal. Increase training load" **Rule: Monthly training load is under 65% of MTL 3.0 |
| 2 | Aerobic Low Shortage | "Total amount of high intensity training is too high with respect to amount of low intensity training. Increase amount of low intensity training to get your training into better balance and to develop performance optimally" |
| 3 | Aerobic High Shortage | "Proportion of aerobic high intensity training has been very low during past 4 week period. Training may not therefore develop aerobic capacity (LT/VO2max) optimally" |
| 4 | Anaerobic Shortage | "Proportion of anaerobic high intensity training has been very low during past 4 week period. As variation in training stimuli per se and impact for anaerobic capacity and speed has been too low - training benefits is expected to be suboptimal" |
| 5 | Balanced | "Your training is in good balance thus expectedly resulting in comprehensive benefit for all areas of fitness" |
| 6 | Aerobic Low Focus | "Your training focus has been in aerobic low intensity training. This type of focus builds foundation for sustaining harder training periods." |
| 7 | Aerobic High Focus | "Your training focus has been aerobic high intensity training. This type of focus typically rapidly improves LT, VO2max and endurance performance." |
| 8 | Anaerobic Focus | "Your training focus has been anaerobic high intensity training. This type of focus maximizes endurance performance rapidly. Notice that your body needs aerobic training focus after this kind of training period". |
| 9 | Above targets, Aer. Low Focus | Your training load is high. Remember to include lighter training periods to your training schedule. |
| 10 | Above targets, Aer. High Focus | |
| 11 | Above targets, Anaerobic Focus | **Rule: Focus is selected for that category which is proportionally closest to "upper-limit" or "very high limit". |
| 12 | Approaching targets** | "Your long term training load is below optimal but is moving towards targets" **Rule: "Approaching target" is provided instead if "below targets" if WTL ≥ 2.5 |

The above table 5 may be modified without departing the scope of this invention. For example, distribution feedbacks #0-8 may form a basic set of feedbacks. Additionally, feedbacks #9-11 may be combined under a generic "Above targets" feedback. Furthermore, feedback #12 "Approaching targets" may be included if a more positive "tone of voice" is preferred instead of corrective feedback.

Table 6 shows the feedback phrase selection logic based on the relationship between the aerobic training load and its related training limits, and the anaerobic training load and its related training limits.

In addition to the typical feedback, some cells in the below table 6 include additional feedback, specifically "(Below targets)" and "(Above targets)". These references may be optionally used to overrule the primary feedback of the MTL based rules presented in table 5. Hence, in certain situations, an additional feedback or rule may be included to overrule the initial rule in particular circumstances.

Table 6: Training load distribution feedback phrase logic

| Aer. low below minimal limit | Aer. High below minimal limit | Aer. High below target | Aer. High in target | Aer. High above target | Aer High above Very high limit |
|---|---|---|---|---|---|
| **Anaerobic below minimal limit** | #5 Balanced (#1 Below targets) | #2 Aer. Low Shortage (#1 Below targets) | #2 Aer. low shortage (#1 Below targets) | #2 Aer. low shortage | #2 Aer. low shortage |
| **Anaerobic below target** | #2 Aer. Low Shortage (#1 Below targets) | #2 Aer. Low Shortage (#1 Below targets) | #2 Aer. low shortage | #2 Aer. low shortage | #2 Aer. low shortage |
| **Anaerobic in target** | #2 Aer. Low Shortage (#1 Below targets) | #2 Aer. low shortage | #2 Aer. low shortage | #2 Aer. low shortage | #2 Aer. low shortage |
| **Anaerobic above target** | #2 Aer. low shortage (#1 Below targets) | #2 Aer. low shortage | #2 Aer. low shortage | #2 Aer. low shortage | #2 Aer. low shortage |
| **Anaerobic above very high limit** | #2 Aer. low shortage | #2 Aer. low shortage | #2 Aer. low shortage | #2 Aer. low shortage | #2 Aer. low shortage (Above targets) |
| **Aer. low below target** | **Aer. High below minimal limit** | **Aer. High below target** | **Aer. High in target** | **Aer. High above target** | **Aer High above Very high limit** |
| **Anaerobic below minimal limit** | #6 Aer. Low Focus (#1 Below targets) | #5 Balanced (#1 Below targets) | #4 Anaerobic Shortage | #2 Aer. low shortage | #2 Aer. low shortage |
| **Anaerobic below target** | #5 Balanced (#1 Below targets) | #2 Aer. Low Shortage (#1 Below targets) | #2 Aer. low shortage | #2 Aer. low shortage | #2 Aer. low shortage |
| **Anaerobic in target** | #3 Aer. high Shortage (#1 Below targets) | #2 Aer. low shortage | #2 Aer. low shortage | #2 Aer. low shortage | #2 Aer. low shortage |
| **Anaerobic above target** | #3 Aer. high Shortage | #2 Aer. low shortage | #2 Aer. low shortage | #2 Aer. low shortage | #2 Aer. low shortage |
| **Anaerobic above very high limit** | #3 Aer. high Shortage | #2 Aer. low shortage | #2 Aer. low shortage | #2 Aer. low shortage | #2 Aer. low shortage (Above targets) |
| **Aer. low in target** | **Aer. High below minimal limit** | **Aer. High below target** | **Aer. High in target** | **Aer. High above target** | **Aer High above Very high limit** |
| **Anaerobic below minimal limit** | #3 Aer. high Shortage (#1 Below targets) | #4 Anaerobic Shortage (#1 Below targets) | #4 Anaerobic Shortage | #4 Anaerobic Shortage | #4 Anaerobic Shortage |
| **Anaerobic below target** | #3 Aer. high Shortage (#1 Below targets) | #3 Aer. high Shortage | #5 Balanced | #7 Aer. High Focus | #7 Aer. High Focus |
| **Anaerobic in target** | #3 Aer. high Shortage | #5 Balanced | #5 Balanced | #7 Aer. High Focus | #7 Aer. High Focus |

(continued)

| Aer. low in target | Aer. High below minimal limit | Aer. High below target | Aer. High in target | Aer. High above target | Aer High above Very high limit |
|---|---|---|---|---|---|
| **Anaerobic above target** | #3 Aer. high Shortage | #8 Anaerobic Focus | #8 Anaerobic Focus | #7 Aer. High Focus | #7 Aer. High Focus (Above targets) |
| **Anaerobic above very high limit** | #3 Aer. high Shortage | #8 Anaerobic Focus | #8 Anaerobic Focus | #8 Anaerobic Focus (Above targets) | #8 Anaerobic Focus (Above targets) |
| **Aer. low above target** | **Aer. High below minimal limit** | **Aer. High below target** | **Aer. High in target** | **Aer. High above target** | **Aer High above Very high limit** |
| **Anaerobic below minimal limit** | #3 Aer. High Shortage (#1 Below targets) | #4 Anaerobic shortage | #4 Anaerobic Shortage | #4 Anaerobic Shortage | #7 Aer. High Focus (Above targets) |
| **Anaerobic below target** | #3 Aer. High Shortage | #6 Aer. Low Focus | #6 Aer. Low Focus | #5 Balanced | #7 Aer. High Focus (Above targets) |
| **Anaerobic in target** | #3 Aer. High Shortage | #6 Aer. Low Focus | #6 Aer. Low Focus | #5 Balanced | #7 Aer. High Focus (Above targets) |
| **Anaerobic above target** | #3 Aer. High Shortage | #6 Aer. Low Focus | #6 Aer. Low Focus | #2 Balanced (Above targets) | #7 Aer. High Focus (Above targets) |
| **Anaerobic above very high limit** | #3 Aer. High Shortage | #8 Anaerobic Focus | #8 Anaerobic Focus (Above targets) | #8 Anaerobic Focus (Above targets) | #8 Anaerobic Focus (Above targets) |
| **Aer. low above very high limit** | **Aer. High below minimal limit** | **Aer. High below target** | **Aer. High in target** | **Aer. High above target** | **Aer High above Very high limit** |
| **Anaerobic below minimal limit** | #3 Aer. High Shortage | #4 Anaerobic Shortage | #4 Anaerobic Shortage | #4 Anaerobic Shortage (Above targets) | #3 Aer. Low focus (Above targets) |
| **Anaerobic below target** | #3 Aer. High Shortage | #6 Aer. Low Focus | #6 Aer. Low Focus (Above targets) | #6 Aer. Low Focus (Above targets) | #6 Aer. Low Focus (Above targets) |
| **Anaerobic in target** | #3 Aer. High Shortage | #6 Aer. Low Focus | #6 Aer. Low Focus (Above targets) | #6 Aer. Low Focus (Above targets) | #6 Aer. Low Focus (Above targets) |
| **Anaerobic above target** | #3 Aer. High Shortage | #6 Aer. Low Focus (Above targets) | #6 Aer. Low Focus (Above targets) | #6 Aer. Low Focus (Above targets) | #7 Aer. High Focus (Above targets) |
| **Anaerobic above very high limit** | #3 Aer. High Shortage (Above targets) | #8 Anaerobic Focus (Above targets) | #8 Anaerobic Focus (Above targets) | #8 Anaerobic Focus (Above targets) | #8 Anaerobic Focus (Above targets) |

A person skilled in the art may modify the above presented 5 x 5 x 5 "decision cube" without departing the scope of the invention. The above presented decision cube may be replaced with a more simple logic, for example using a 3 x 3 x 3 logic cube where each intensity category may be determined with 3-level scale: 1) below target 2) in target and 3) above target. The embodiment represented in the table above, including the 5-level scaling where each level may be as follows - 1) below minimal limit 2) above minimal limit but below target 3) in target, 4) above target and 5) above very high limit - may enable more precise feedback while a more simple system may be easier to visualize in devices having small displays where additional limits may not fit that well to the device display.

As described above, training load distribution may be shown per each label or alternatively by dividing labels e.g. into 3 groups: Labels 1-2 to *aerobic low load* category, labels 3-5 to *aerobic high load* category and labels 6-7 to *anaerobic load* category. Individual labels may be named as follows: label 1 for "Rest/Recovery", label 2 for "Base", label 3 for "Tempo", label 4 for "Lactate Threshold", label 5 for "VO2max", label 6 for "Anaerobic Capacity" and label 7 for "Speed". Optionally, there may also be a label 0, which represents "Rest" only.

Each workout may accumulate both *aerobic* and *anaerobic load.* Aerobic load units are transferred to selected aerobic label and *anaerobic load* units are transferred to selected anaerobic label. As an example, if workout's aerobic load has 75 units and workout label is 2 and workout's *anaerobic load* has 25 units and workout label is 7, then the 75 units of aerobic load are transferred to aerobic Base -label (label 2) and 25 units of load are transferred to aerobic Speed -label (label 7). Units may be ml/kg, when aerobic load is based on EPOC, or different arbitrary units, when aerobic load is based on TRIMP.

Figure 1 shows an exemplary flowchart for providing a next workout recommendation. At a start, sleep score of a user may be evaluated.

**Sleep score**

Sleep score may be based on a value representing both a sleep duration and a sleep quality score. Sleep score may be age dependent. These variables are determined based analysis of heart rate variability (HRV) and, optionally, acceleration data, during identified sleep periods. Sleep score may be calculated based on a weighted sum of scaled values for optimal sleep duration and sleep quality. Sleep quality may relate to an amount of sleep at different sleep stages and restlessness of the sleep.

There are many well-known methods of identifying the beginning and end of sleep periods based on heart rate or HRV, which allow for the measurement of sleep duration. Because different ages require different amounts of sleep, the sleep duration may be assessed in comparison to age-based recommended sleep durations. Optimal sleep duration may be based on age-dependent individual sleep duration demand. Demand for sleep may increase, for example, due to sleep debt, stress and/or strenuous workout.

Sleep quality is based on a weighted scoring of overnight stress and recovery, an analysis of the proportion of the identified sleep stages during sleep, both of which are measured using continuous monitoring of heart rate and HRV, and a measure of sleep restlessness. Sleep stress and recovery can be determined through continuous monitoring of heart rate and heart rate variability via, for example, a wrist-worn PPG device. Identified periods of elevated heart rate representing stress or exercise may increase a user's cumulative stress levels, and identified periods of lower heart rate may represent recovery, even during sleep periods.

Using wrist-worn PPG heart rate monitoring, it is well-known that a user's sleep stages may identified. These include sleep states of "deep sleep", "REM sleep", and "light sleep", in addition to an "awake" state. A weighted average of the proportion of each sleep state in a night comprise this element of the sleep quality score, where, for example, a night with a relatively high proportion of deep sleep will produce a low high score, and a night with a high proportion of light sleep, awake time, or REM sleep may produce a low score. These values are in line with well-known values on what proportions of types of sleep represent optimal sleep periods. Optimal sleep quality may include less than 60% of light sleep, more than 20% deep sleep and 20-30% REM sleep.

Restlessness may be measured using accelerometer data. This may be measured by, for example, measuring the number of "short" periods of immobility, short being, for example, one minute or less. Fewer short periods of immobility would suggest lower restlessness and higher sleep quality. The result of these measurements over an entire night may be scaled against database percentile results. A final sleep quality value can then be calculated using a weighted average of these variables.

The final sleep score, based on both sleep duration and sleep quality may be scaled, for example, to a score from 0 to 100, where 100 is an optimal sleep score.

**NWR embodiment based on Figure 1**

If a sleep score is, for example, under 25, it is determined as very poor sleep quality. If the sleep is determined as very poor 111, rest or recovery 101 is recommended as the NWR. Otherwise next evaluations are made at phase 112 in order to provide the NWR.

At the next phase 112 the sleep score, the weekly training load, the recovery time, the training status TS, the weekly training target and the number of successive training days are examined. The parameters may be examined in different order.

If a sleep score is determined to be, for example, 25-40, it is determined as moderate. At this phase, after the previous examination of sleep score, it is compared, whether the sleep score is under 40. if yes, the sleep score is determined to be moderate. In this case a scheduled workout for today is replaced with an easier one. For example, if the scheduled workout was hard, it is replaced by easy base training 102, and if the scheduled workout was easy, it is replaced by recovery 101.

The weekly training load WTL may be based on detected training load peak values from performed trainings during the past week. The present WTL describes amount of training the user has performed during the past week, which means one week from today including today. If WTL is detected to be close to WTL target, recommendation is to rest or an active recovery 101.

If recovery time is over 36 hours, it is determined as pretty high recovery time. Due to this, a harder workout is replaced by an easier one. If the determined recovery is high and WTL target is approaching, a Base training 102 is recommended.

The training status determination takes into account various aspects of a user's training history, such as their weekly training load, monthly training load, changes in fitness level, or recovery test values or similar variables that describe a user's training history, fitness level, or recovery state. Exemplary methods for determining training status are described in the applicant's own patent publication US10580532.

If a training status describes a state that may be described by the term "overreaching", the user has been training hard enough that the body needs extra time to recover, and the next recommendation is to focus on rest or active recovery at phase 101. Depending on the goal of training, rest period or easy training period can be recommended either immediately or just after a few days of detected a state that may described "overreaching". For example, for an average active user, a rest or easy training period may be triggered on the very first day of "overreaching" whereas high level athletes may be allowed to stay in the state that may be described "overreaching" even for a few days. The amount of successive rest or active recovery days may be limited in a way that for example after two such easy days at least a base workout is recommended regardless of staying in that state that may be described "overreaching" at phase 102. This is due to preferring Base training instead of resting many days without training.

Number of successive training days is examined. The amount of allowed successive training days is dependent on the activity class of the user but also depends on that detected training status. For example, a training state that may be described by the term "overreaching" may be an exception where preferably rest and easy training is prescribed. In that case, even multiple successive rest days may be prescribed in order to avoid development of overtraining state. In all other states the load is regarded not to exceed the user's personal training tolerance - unless poor sleep or single very demanding exercise challenges user's capability to recover. If these exception cases are omitted, rest or active recovery training day can be prescribed if a workout label 2-7 has been achieved every day on days-1 to days-(AC/2+1); wherein days-1 is yesterday (today minus one day) and AC is user's activity class varying e.g. from 4 to 10, and days-(AC/2+1) thereby 3-6 days in past. As the description above points out, for example, poor sleep, moderate sleep or a single exercise causing high recovery time may further limit the amount of allowed successive training days. Furthermore, the amount of successive training days may also independently be limited by the load on previous 4-14 days, for example 7 days, especially if the weekly training load WTL is already very close or even above the target load of current week. Detecting "overreaching" on multiple successive days in the training history leads to recommendation of Base training.

As said above, if WTL is close to the WTL target, a recovery 101 or Base training 102 is recommended. This can be checked, for example, by analyzing the load of last 6 days and supplementing this load value with a load corresponding to training effect 2.5. If performing training effect 2.5 today would cause exceeding the WTL target of current week, then either rest or active recovery may be recommended for today. This may still be dependent on training effect because if WTL is close to the WTL target, but there are 1-2 past successive days detected as recovery, Base training 102 may be recommended to avoid resting many days in a row.

To summarize, rest or active recovery recommendation 101 for today may be triggered by many trigger mechanisms independently. For example, both exceeding allowed number of successive training days or exceeding weekly training load target may be independent trigger mechanisms.

Ideally, next workout recommendation can be asked/called at any time during a day and recommendation is based on data measured from the user. That is, the target is to include all stress factors affecting the user to improve decision-making considering what exercise prescription would be optimal at any time. Accordingly, in addition to measured sleep quality and awake stress levels, workout recommendation should change also with respect to timed and non-timed activities performed earlier on that day. The amount of timed activities may be measured using accumulated training load. If daily limit value has been exceeded then workout recommendation for the remainder of that day may be for example "rest or active recovery". A workout may be regarded as completed if the accumulated load corresponds to a training effect value of 2.5. Alternatively, distance travelled may also be set as a threshold for determination whether a workout is already completed or not. For example, running distance over 5 km or biking distance over 20 km could be used as such limit values. These distances may be set differently based on, for example, activity class, or personal training history.

If the previous examinations are found negative, the examination continues to the next phase 113, where amount of *aerobic high load* is examined. *Aerobic low load* may generally refer to low-intensity aerobic training, for example aerobic exercise at a HR below 80% of a user's maximum HR. *Aerobic high load* may generally refer to exercise involving a higher HR than a defined intensity for HR low, but it does not belong to *anaerobic* category, and therefore, the VO2max limit is not exceeded. Anaerobic training is performed at intensities beyond a user's VO2max. Training history data may comprise load sums and load target ranges for each intensity category. Range limits for each different intensity categories may be determined based on user's monthly target loads and user's activity class AC.

*Aerobic high load* training intensities can be used to optimize aerobic capacity. However, regardless of being efficient in optimizing aerobic (cardiorespiratory) capacity this kind of training increase training load rapidly and can thus not be repeated as often as *aerobic low load* or Base workouts. Accordingly, aerobic low intensity training allows training on a daily basis (or even several daily workouts) in long term which is why this type of training forms the basis of endurance training. Anaerobic training is performed at intensities beyond a user's VO2max. They are needed to optimize performance as this kind of training improves, for example, exercise economy, as well as capability to (repeated) sprints which are crucial characteristics in endurance sports.

During the next steps 113 and 115 it is detected whether enough *aerobic high load and anaerobic load* training has been performed during the previous four weeks as well as during the last few days, for example previous 1-7 days. Accordingly, Different timely windows are needed in the decision making considering the next planned workout. Firstly, it is important that the algorithm checks that the last month includes a suitable amount of high intensity effort, e.g. as a percentage of training load target, as shown in previous tables 5 and 6. If the amount of high intensity effort is very high in a 4-week history then the algorithm should preferably prescribe *aerobic low load* workouts until a more balanced monthly distribution is achieved but still keeping at least one *aerobic high load* workout in a weekly plan. Accordingly, it is important to keep the amount of high intensity training adequate in a 7-day window. In this way imbalances in training load distribution can be fixed faster than using a preplanned workout template but simultaneously avoiding situation where the user gets, for example, base workouts successively for a week or more (e.g. a case where monthly load distribution shows *aerobic low* shortage). Furthermore it is also important to check that the last few workouts do not include too much of high intensity effort. This helps in keeping the planned workouts reasonable in a situation where, for example, a monthly load distribution is balanced and thus would allow performing any kind of workouts in short term.

In an exemplary embodiment there must be at least two consecutive easy (label 1 or label 2) workouts before any *aerobic high load* workouts can be prescribed. If enough *aerobic high load* training has been performed 113 and there is enough *anaerobic load* recently, Base workout 102 is recommended next. If previous days don't comprise any *aerobic high load* workouts, then an *aerobic high load* workout is recommended as the next recommended workout. If either one of the 2 previous workouts includes an *aerobic high load* workout and if *anaerobic load* level is low, and if the previous workout label has been either label 1 or label 2 then the next recommended workout may be an Anaerobic Capacity or Speed workout. As the names of the workouts already describe, these *anaerobic load* category workouts are intended to improve anaerobic and sprinting performance both of which are very important, for example, considering competitive performance, also in endurance sports. Accordingly, next phase may be to examine the label distribution and optionally the rhythm between the Anaerobic Capacity and Speed workouts performed in the history as both training types (labels) are important as such. Ratio of Anaerobic Capacity 106 and Speed training 107 may be determined, and compared, for example, to a target load ratio of 70:30 (Capacity:Speed), and the next workout recommendation may be selected in accordance to achieving or maintaining that predetermined 70:30 ratio.

If it is determined that previous days don't include enough *aerobic high load* training, meaning that examination 113 is negative, next label distribution and optionally the rhythm in the training history is determined 114. *Aerobic high load* workouts are intended to improve aerobic capacity and they include Tempo 103, VO2max 104 or Lactate Threshold LT 105 workout labels. As all of these workout types (labels) are important as such in optimizing the development of aerobic capacity, a target ratio between these labels is preferably calculated. Target ratio of Tempo, VO2max and LT training may be divided, for example as 35% of Tempo, 20% of VO2max, 45% of LT in terms of load accumulated during such workouts and summing up to 100% of *aerobic high load.* The next workout recommendation may be selected in accordance to the predetermined ratio.

Examining label distribution and rhythm in the training history enables maintaining balance in training and distributing different kind of workouts effectively. This enables development towards the training goal. This avoids overtraining and possible injuries due to such. Taking into account different training intensity categories, their relations and distribution, enables efficient development according to the training load target and training goal.

Examination of training history data enables optimizing long term targets. This is taken into account for the next workout recommendations NWR. Findings triggering corrective recommendations may include:

- Monthly training load MTL being under 65% of MTL3.0, which is determined to be below target;

- Total amount of *aerobic high load* may be determined too high with respect to amount of total training load;
- *aerobic low load* being too low with respect to amount of total training load;
- Proportion of *aerobic high load* being too low with respect to amount of total training load during last week or last 4 weeks;
- Proportion of *anaerobic load* being very low during several past weeks;
- Training load is high.

As explained above, these corrective recommendations may change the week level workout prescriptions significantly but on the other hand do not necessarily cause omission of any intensity category totally. For example, if the user's 4-week training load distribution has too much of *aerobic high load,* then the proportion of *aerobic low load* workouts is increased but still keeping at least one *aerobic high load* workout in the weekly prescription window.

Taking into account the history data enables providing productive recommendations. For example, doing too much, too long, at too high pace or alike situations may be detected based on the training history data.

Based on evaluation, as illustrated in Fig. 1, next workout recommendation NWR is provided. NWR includes recommendation for the next exercise to be performed. NWR may provide a detailed structure for the next exercise including, for example, a warm-up, repeats and a cooldown. Training guidance for the next week may be provided. This means that NWRs up to the following 7 days, or more, may be provided. For example, it is possible to provide three optional training guidance: general training guidance, running specific training guidance and cycling specific training guidance. Generic workout recommendations may be useful for a user who is just a beginner. In such case, recommendations are preferably more simple than the running or cycling specific recommendations. Generic workout structures are kept more simple by preferring steady paced workouts instead of interval workouts, for example. In addition, it is possible to exclude the most complex workouts totally from the generic workout recommendations. For example, Speed and Anaerobic Capacity workouts may be excluded, since they are not essential for a beginner.

A user interface may be providing all next workout recommendations NWRs even in a single user device in a way that generic workout recommendation can be regarded as a generic cardio training plan. In addition to that running and cycling specific training recommendations may be provided.

Running and cycling specific workout recommendations are often targeted for more advanced users. The recommendations may include more complex workout structures, for example with multiple sets of intervals. Different structures help achieving various training goals. On the other hand, they may be too complex for beginner to understand.

Another user interface embodiment may be providing only user specific next workout recommendations NWR in a single user device in a way that only generic, running or cycling workout recommendation is be provided.

**NWR Input**

Inputs are used for determining one or more following next workout recommendations NWRs. Recovery time is one input and only easy Base training is recommended if examined recovery time is high. Sleep score may be used as an input. After a poor quality or low amount of sleep, only easy Base training is recommended. Weekly training load WTL is calculated based on training history data. When WTL is close to the WTL target, only easy Base training is recommended. *Aerobic high load* workouts are recommended when there is enough gap for WTL to increase. Based on the gap between the current WTL and WTL target, easier or harder workout option is recommended, where possible. Training load focus is calculated based on training history data. Training rhythm is fine tuned if the user has shortage in some of the intensity categories. In this case, target is not to fix the shortage immediately, but rather to do so in a reasonable manner, typically over a longer period of time and multiple workouts. Training status is calculated based on training history data. Only rest or recovery is recommended if training state may be described by the term "overreaching". Rhythm of workouts may be divided by defining ratios or amounts for different kind of workouts. This may be dependent on primary benefit or label that is achieved from a certain workout. Highest amount of successive training days is determined based on user's activity class. Generally, two Base workouts are placed between *aerobic high load* workouts, where one of the Base workouts may be an anaerobic or sprint type of workout, provided that the *anaerobic load* remains below the target level in the WTL.

When *aerobic high load* workouts are recommended, current proportion of training loads of possible options, being Tempo, LT and WO2max, is taken into account. Training loads may be divided between as targets, e.g. Tempo 20%, LT 50% and VO2max 30%.

Labels or workout labels may be regarded as indicators of primary benefits of the workouts. Workouts may also have secondary labels or secondary benefits. For example, if the primary label (benefit) is from the *aerobic low load* or *aerobic high load* categories (Recovery, Base, Tempo, Lactate Threshold or VO2max), then the workout may still have a secondary label from the *anerobic load* category of labels (Anaerobic Capacity or Speed) as the secondary benefit. Vice versa, the workout may also have Anaerobic Capacity or Speed as the primary benefit and consequently Recovery, Base, Tempo, Lactate Threshold or VO2max as the secondary benefit.

Monthly or weekly intensity category distribution may be updated after each exercise based on the primary and secondary labels. The training load distribution may accumulate corresponding to the primary and secondary labels, or corresponding to the primary labels only.

While the primary energy production pathways of human body can be divided into aerobic and anaerobic and while each workout can develop both of these pathways - common coaching and physiological knowledge suggests that only one aerobic characteristic or category can be improved at the time. It is suggested that it is not effective trying to develop, for example, Base and Lactate Threshold in the same single exercise. Accordingly, in one preferred embodiment of this invention primary and secondary labels (benefits) cannot include labels from the two aerobic categories (*aerobic low load* and *aerobic high load*) but instead one of the two labels (benefits) must be anaerobic. In case the anaerobic training effect remains below 1.0 (which may be typical in steady paced Recovery or Base workouts), then the secondary label (benefit) may not be assigned at all. On the contrary, Anaerobic Capacity and Speed workouts often stress aerobic system to such an extent that aerobic training effect 1.0 is mostly exceeded and thus the secondary label (benefit) is mostly assigned. For example, if a Speed workout comprises walking between the Sprint bouts, then the secondary effect may be Base. On the other hand, if the periods between the sprinting bouts include brisk running then user's cardiorespiratory system may be even heavily taxed and thus the secondary benefit may be Tempo, for example.

**NWR output**

NWR output may comprise a label. Labels may be associated to each workout in order to provide additional feedback. The label provides a description of impacts of a workout covering both aerobic and anaerobic training. The labels are based on aerobic and anaerobic feedback phrases. Each workout may accumulate both *aerobic* and *anaerobic load*. The determined aerobic training load is transferred as a label based on rules for aerobic training; and the determined anaerobic training load is transferred as a label based on rules for anaerobic training. Based on cumulative training load sum for both anaerobic and aerobic training load, the respective training load unit is collected. Over multiple workouts, training load units identify the proportion of the types of training over a given period.

The labels enable analyzing distribution of training load. The distribution may be simplified into coherent intensity categories that generally describe the energy systems being used. Distribution of the training load is based on training loads collected over a month, or extrapolated to represent approximately a month. Training load may be divided into three intensity categories (*aerobic low load, aerobic high load* and *anaerobic load*) based on the workout label of each exercise. *Aerobic low load* may generally be defined as low-intensity aerobic training, for example, aerobic exercise at a heart rate below 80% of a user's maximum heart rate. This kind of training forms the basis of any endurance training plan as this type of training allows high training volumes. *Aerobic high load* would then be considered exercise that involves a higher heart rate than the defined intensity threshold of *aerobic low load* exercise, but does not belong in the category of being an anaerobic exercise. Aerobic high training intensities may be used to optimize aerobic capacity. Anaerobic training is performed at intensities beyond a user's VO2max. They are needed to optimize performance as this kind of training improves, for example, exercise economy, as well as capability for (repeated) sprints which are crucial characteristics in endurance sports.

The labels help in differentiating well-structured training from poorly planned training. For example, traditional "time in zone" analysis may show a good distribution of intensities in a long term analysis even if a user performs high intensity interval training in each workout, since time at low aerobic intensities accumulate during warm-ups and cooldowns. Generally, based on the traditional measures, no major changes are detected as being necessary, nor recommended for future trainings. However, excess amount of high intensity training is actually performed. Thus, one benefit from the labels is to reveal excess amount of high intensity training. This enables maintaining balance in development of body's energy systems by recommending more low intensity workouts.

In addition, a traditional HR based intensity zone model is not able to provide any information on accumulated time or effort at supramaximal intensities. This leads to excluding different kinds of anaerobic training (speed endurance and pure speed) from the overall training load distribution.

NWR output may comprise an estimate on aerobic training effect (aerTE). NWR output may comprise an estimate on anaerobic training effect (anTE). NWR output may comprise a workout structure. The workout structure may comprise periods for warm-up, repeats and cooldown. Warm-up and/or cooldown may comprise duration, distance or training load. Warm-up and/or cooldown may additionally comprise intensity of the workout period, for example in watts, HR or pace. Repeats comprises the effective training period. It may comprise number, duration and intensity of repeats, as well as duration and intensity of recovery bouts. Intensities may be in watts, HR or pace. Different exercise modes, such as cycling or running, influence the body and fitness in slightly different ways. Although every kind of exercise is good and the primary benefits are substantially the same, different exercise modes burden the body differently and their benefits may slightly differ. A fit and experienced cyclist who has not been doing running workouts may not have the capabilities to fully benefit the same running exercise as a dedicated runner of the same activity class, and vice versa. This should be taken into account when planning workouts and providing workout structures.

In an exemplary embodiment the workout structure may be divided into target zones that correspond to workout labels. The zones may be further divided into different exercise specific targets. Heart Rate (HR) zone targets may be defined according to percentage ranges derived from the user's lactate threshold heart rate (LTHR). Bike zone targets may be defined according to percentage ranges derived from the user's functional threshold power (FTP). FTP functions for cyclists in the same way that Lactate Threshold works for runners. It reports the intensity of physical activity above which your body will rapidly fatigue. Run Pace zone targets may be defined according to percentage ranges derived from the user's lactate threshold speed (LT-speed). Table 7 defines one embodiment for the determination of different Zone targets in different exercise modes.

VO2max referred to in table 7 as well as elsewhere in the description may be determined using applicants method described in the publication US10123730.

FTP and LT-speed (lactate threshold-speed) referred in the table 7 as well as elsewhere in the description may be determined using applicants method described in the publications US9517028 and US9693727.

Table 7. Zones for NWR on different exercise modes

| HR zone | Zone name | Target HR |
| --- | --- | --- |
| 1 | Warm-up & Recovery | 65-79%LTHR |
| 2 | Base | 80-89%LTHR |
| 3 | Tempo | 90-95%LTHR |
| 4 | LT & FTP | 95-100%LTHR |
| 5 | VO2max | > 100% LTHR |
|  |  |  |
| **Bike Zone** | **Zone name** | **Target Power** |
| 1 | Warm-up & Recovery | 0-54%FTP |
| 2 | Base | 55-74%FTP |
| 3 | Tempo | 75-89%FTP |
| 4 | LT & FTP | 90-104% FTP |
| 5 | VO2max | 105-119%FTP |
| 6 | Anaerobic (capacity) | 120-150%FTP |
| 7 | Sprint | >150%FTP |
|  |  |  |
| **Run Pace zone** | **Zone name** | **Target speed** |
| 1 | Warm-up & Recovery | 0-75% LT_speed |
| 2 | Base | 76-91%LT_speed |
| 3 | Tempo | 92-96%LT_speed |
| 4 | LT & FTP | 97-102%LT_speed |
| 5 | VO2max | 103%LT_speed*--> (VO2max-3.5)/3.33 km/h |
| 6 | Anaerobic (capacity) | x% vVO2max = x* (VO2max-3.5)/3.33 km/h |
| 7 | Sprint | x% vVO2max = x* (VO2max-3.5)/3.33 km/h OR Step rate > 210 steps/min |

In case the information on LTspeed is missing it may be determined using information on user's maxMET (=VO2 Max/3.5) as follows:

$$LTspeed\ (m/s) = (maxMET*3.5-3.5)/12*0.828 + 0.1486$$

Similarly, in case information on FTP is missing it may be determined using information on user's maxMET (=VO2 Max/3.5) as follows:

$$FTP\ (W) = ((maxMET*3.5*weight-350)/12.24)*0.828$$

Since user's personal maximal heart rate is reached at the speed that corresponds to users VO2 Max, it may be difficult to define exact heart rate limits for anaerobic capacity and speed workouts. However, as heart rate limits may have some value at some exercise modes (for example skating where target speed may be harder to estimate), target heart rates may be optionally defined for these label 6 and label 7 workouts. Determining target HR for supramaximal workouts is directional rather than normative. Directional limits may be the following:

Target HR for Label 6 workouts is >= 95%LTHR
Target HR for Label 7 workouts is >= 90%LTHR

Label 6 workouts typically include longer intervals than label 7 workouts, which is why the directional HR is higher in label 6 workouts.
Next workout recommendation may be provided using a workout structure, which includes at least one or more of the following information:

- label and the corresponding workout phrase
- duration of the NWR
- running or cycling distance
- training effect
- anaerobic training effect
- number of interval repeats
- number of set repeats
- workout profile** warm-up
- workout profile** work
- workout profile** interval rest
- workout profile** set rest
- workout profile** cooldown

The above workout profiles (**) may comprise the following information: VO2max (min/max), HR (min/max), speed (min/max), power (min/max), and/or duration.
Specific target values may be set for %VO2max (min/max), HR for example as bpm(min/max), speed for example as km/h or as m/s (min/max) and/or power for example as watts (min/max), duration for example as seconds, or as minutes in the above workout profiles.
Heart Rate zone workout may be a generic workout. Bike zone workout may be a cycling workout. Run Pace workout may be a running workout.
The workout profile may include at least one or more intensity ranges and target duration for each workout phase.
Each planned workout may have a default warm-up and cooldown duration and intensity. Default duration of warm-ups and cooldowns may be between 10 and 15 minutes, for example. The prescribed intensity zone for the warm-ups and cooldowns may be either zone 1 or zone 2. The intensity during actual work period between warm-up and cooldown depends on the target label (benefit) of the workout. Typically, if the goal of the workout is label 2, that is, the target is to improve base endurance, then zone 2 intensity is prescribed. Furthermore if the target is to achieve label 3, 4, 5, 6 or 7 then zones 3, 4, 5, 6 or 7 are used as the prescribed intensity, respectively.
The label 2 (base endurance) workout structure between warm-up and cooldown is typically a steady pace workout structure so that the target intensity range remains constant during that work period. It is even possible to omit warm-ups and cooldowns from these workouts in order to keep that workout structure simpler since there is no need from a physiological point of view for warm-ups and cooldowns as the effort level is relatively low and, for example, blood lactate levels remain low during such a workout. As all individuals perform the workout with somewhat similar relative intensity (80-89%LTHR) the duration of workout largely depends on two factors: 1) user's activity class and 2) the exact training effect level planned for the workout. Since the target training effect in all of the labels may have a predetermined range - meaning a range from 2.5 to 3.7 for example in label 2 workouts - it means that the durations tend to increase as the activity class of a user increases. Of course, also extending target training effect from 2.5 to 3.7 extends the duration of a workout significantly. Accordingly as the duration may increase as a function of both active class and the target training effect duration of planned label 2 workouts may vary from 20 min (easiest workout of a user with low activity class) up to 150 min (hardest workout of a user with high activity class), for example.

Label 3-7 workouts may have fixed duration for warm-ups and cooldowns. The work period between warm-up and cooldown may now include several repeats each repeat including a short post-repeat-recovery-period. Furthermore, repeats may be allocated into several sets where each set may have a prolonged post-set-recovery-period. If these kind of label 3-7 workouts have several repeats or sets they are always scaled in a way that a user with a lower activity class performs a significantly lower amount of repeats and/or sets than a user with a high activity class. Also the duration of each repeat may be shorter for a user with lower activity class. Accordingly, as the duration of repeats, the number of repeats and the number of sets can all be scaled it means that any workout structure can now be scaled in order to be suitable workout for both a beginner and even an advanced athlete. For example, a tempo workout may have following structure:

10 min Warm-up at zone 1 + 3-5 x 8-10 min at zone 3 followed by 5 min recovery at zone 1 between intervals + Cooldown 15 min at zone 1. In this kind of workout number and duration of repeats are so that target aerTE (e.g. aerTE 3.8) and label 3 will be reached after the whole workout. That means that beginner will only do 3 x 8 min repeats whereas a high level athlete will do 5 x 10 min repeats meaning that the duration of high intensity work can be doubled while user develops from a beginner to advanced level.

The target training effect range of label 1 workouts is lowest and maybe from 1.5 to 2.2 for example. The target training effect range of label 2 workouts may be from 2.5 to 3.7, for example. The target training effect of label 3 label 4 and label 5 workouts maybe from 3.8 to 4.2, for example. The target training effect of label 6 workouts maybe from 3.0 to 3.3. the target training effect of label 7 workouts maybe from 2.5 to 2.8, for example.

Since the workout structure is now fixed for each workout in terms of target training effect (aerTE and anTE) and planned intensity profile, the target work duration between warm-ups and cooldowns as well as number and duration of repeats and number of sets may be determined based on a simulation where a target intensity is input into applicants training effect algorithm (See applicant's publications US7805186B2, US8052580B2, US8465397B2). Based on the results of one or multiple simulations a workout structure providing closest match with the target aerobic training effect, target anaerobic training effect, and the target label may be selected. This simulation approach may be used in all workouts. Alternatively, it is also possible to use the simulation approach only in steady pace workouts whereas the selection of suitable interval workout structure is done based on a multivariate function (e.g. linear interpolation). Simulation may provide more accurate results but by using a mathematical function especially in interval workouts the use of calculational power resources may be reduced significantly. On the other hand, determining the target duration based on information on target training effect is relatively resource efficient in steady pace workouts as only one simulation is needed to find the optimal duration. As a user with ordinary skilled in art may understand, especially in label 2 workouts, additional rules may be used considering the target duration of a workout. For example, if current day is a working day, then a lower range of allowed durations may be used when compared to Saturday or Sunday. For example, the duration of planned workouts may be forced between 20 to 90 minutes during working days whereas duration may be extended up to 150 minutes during weekends.

Table 8 shows examples about how the exact workout structure may be selected. Each exercise may have a different detailed warm-up, exercise and cooldown structure. Values of aerTE and anTE may get values different from example limit values of the table 6. For certain values of aerTE and anTE, multiple options may exist, as is shown in the table 6 and the following description.

Table 8: Workout structure and example target aerTE and anTE values in different label workouts and different exercise modes

| Cycling workouts | | | |
|---|---|---|---|
| Workout label | aerTE | anTE | Exercise structure |
| Recovery (recovering exercise) | 1.5-2.2 | 0.0 | Exercise |
| Recovery (cool down) | 1.5 | 0.0 | Exercise |
| Base (AC>=7) | 2.5-3.7 | 0.0 | warm-up Exercise1 Cooldown |
| Base (AC<7) | 2.5-3.7 | 0.0 | warm-up Exercise2 Cooldown |

(continued)

| Cycling workouts | | | |
|---|---|---|---|
| **Workout label** | **aerTE** | **anTE** | **Exercise structure** |
| Tempo | 3.8-4.2 | 2.0 | warm-up Exercise1 Cooldown |
| Tempo | 3.8-4.2 | 2.0 | warm-up Exercise2 Cooldown |
| FTP & LT | 3.8-4.2 | 2.0 | warm-up Exercise1 Cooldown |
| FTP & LT | 3.8-4.2 | 2.0 | warm-up Exercise2 Cooldown |
| VO2max | 3.8-4.2 | 2.0 | warm-up Exercise1 Cooldown |
| VO2max | 3.8-4.2 | 2.0 | warm-up Exercise2 Cooldown |
| Anaerobic | 3.0-3.3 | 3.0-3.3 | warm-up Exercise1 Cooldown |
| Anaerobic | 3.0-3.3 | 3.0-3.3 | warm-up Exercise2 Cooldown |
| Anaerobic | 3.0-3.3 | 3.0-3.3 | warm-up Exercise3 Cooldown |
| Anaerobic | 3.0-3.3 | 3.0-3.3 | warm-up Exercise4 Cooldown |
| Speed | 2.5-2.8 | 2.5-2.8 | warm-up Exercise1 Cooldown |
| Speed | 2.5-2.8 | 2.5-2.8 | warm-up Exercise2 Cooldown |
| **Running workouts** | | | |
| **Workout label** | **aerTE** | **anTE** | **Exercise structure** |
| Recovery (recovering exercise) | 1.5-2.2 | 0.0 | Exercise |
| Recovery (cool down) | 1.5 | 0.0 | Exercise |
| Base (VO2max < 55) | 2.5-3.7 | 0.0 | Exercise |
| Base (VO2max >= 55) | 2.5-3.7 | 0.0 | warm-up Exercise |
| Tempo-interval | 3.8-4.2 | 2.0 | warm-up Exercise1 Cooldown |
| Tempo-interval | 3.8-4.2 | 2.0 | warm-up Exercise2 Cooldown |
| FTP & LT | 3.8-4.2 | 2.0 | warm-up Exercise1 Cooldown |
| FTP & LT | 3.8-4.2 | 2.0 | warm-up Exercise2 Cooldown |
| FTP & LT | 3.8-4.2 | 2.0 | warm-up Exercise3 Cooldown |

(continued)

| Running workouts | | | |
|---|---|---|---|
| **Workout label** | **aerTE** | **anTE** | **Exercise structure** |
| VO2max | 3.8-4.2 | 2.0 | warm-up Exercise1 Cooldown |
| VO2max | 3.8-4.2 | 2.0 | warm-up Exercise2 Cooldown |
| Anaerobic | 3.0-3.3 | 3.0-3.3 | warm-up Exercise1 Cooldown |
| Anaerobic | 3.0-3.3 | 3.0-3.3 | warm-up Exercise2 Cooldown |
| Anaerobic | 3.0-3.3 | 3.0-3.3 | warm-up Exercise3 Cooldown |
| Speed | 2.5-2.8 | 2.5-2.8 | warm-up Exercise1 Cooldown |
| Speed | 2.5-2.8 | 2.5-2.8 | warm-up Exercise2 Cooldown |
| **Generic running and cardio workouts** | | | |
| **Workout label** | **aerTE** | **anTE** | **Exercise structure** |
| Recovery (recovering exercise) | 1.5-2.2 | 0.0 | Exercise |
| Recovery (cool down) | 1.5 | 0.0 | Exercise |
| Base | 2.5-3.7 | 0.0 | Exercise |
| Tempo | 3.8 | 2.0 | warm-up Exercise Cooldown |
| FTP & LT | 3.8 | 2.0 | warm-up Exercise Cooldown |
| **Generic cardio workouts** | | | |
| **Workout label** | **aerTE** | **anTE** | **Exercise structure** |
| Recovery (recovering exercise) | 1.5-2.2 | 0.0 | Exercise |
| Recovery (cool down) | 1.5 | 0.0 | Exercise |
| Base | 2.5-3.7 | 0.0 | Exercise |
| Tempo | 3.8 | 2.0 | Warmup Exercise Cooldown |
| FTP & LT | 3.8 | 2.0 | Warmup Exercise Cooldown |

According to an embodiment the workout structure of label 1-5 workouts for any given exercise mode may contain at least an easy option and a hard option. The easiest option may have shorter duration and/or lower aerobic training effect (aerTE) and/or less repeats than any of the harder workout structures. The hardest option may have longer duration and/or higher training effect (aerTE) and/or more repeats than any of the easier workout structures. The contents of the workout structures in different options may be dependent on the activity class (AC) of the user. The higher the AC, the longer the duration and/or the higher the repeat number is in each option. Label 1-5 workouts may include also a (secondary) anaerobic training effect (anTE) target. AnTE target is typically lower than the aerTE in this kind of workouts. Especially interval-type aerobic workouts may tax anaerobic energy production systems to certain extent as each repeat may start with an acceleration phase that exceeds body's aerobic energy production capacity. Using the secondary target may help the user to understand that it is not harmful to slightly engage also the anaerobic energy pathways in aerobic (interval) training.

According to an embodiment the workout structure of label 6-7 workouts for any given exercise mode may contain at least an easy option and a hard option. The easiest option may have shorter duration and/or lower anaerobic training effect (anTE) and/or less repeats than any of the harder workout structures. The hardest option may have longer duration and/or higher anaerobic training effect (anTE) and/or more repeats than any of the easier workout structures. The contents of the workout structures in different options may be dependent on the activity class (AC) of the user. The higher the AC, the longer the duration and/or the higher the repeat number is in each option. Label 6-7 workouts may have a (secondary) aerobic training effect target. Such (secondary) target may be used to help the user to understand that user will anyway have a certain effect to cardiorespiratory system as well, since all anaerobic workouts stress also human cardiorespiratory system to a certain extent - and especially, it is ok that such aerobic training effect is acceptable as the end result of a workout.

According to an embodiment the easiest Rest and Recovery label workout structure in the Cycling exercise mode may have a target training effect (aerTE) 1.5 and contain an exercise at *Warm-up & Recovery zone* until aerTE 1.5 is reached. The hardest Rest and Recovery label workout in the Cycling exercise mode may have a similar structure, but have a higher target training effect (aerTE), for example 2.2.

According to an embodiment the easiest Base label workout structure in the Cycling exercise mode may have a target training effect (aerTE) 3.0 and contain a 10 min warm-up at *Warm-up & Recovery zone,* an exercise at *Base zone* until aerTE is about 2.8, or such that the whole workout aerTE will reach 3, and a 5 min cooldown at *Warm-up & Recovery zone.* If the activity class of the user is below 7, the warm-up may be 20 min at *Warm-up & Recovery zone* that would allow longer duration before target aerTE (or target load level) is achieved. The hardest Base label workout in the Cycling exercise mode may have a similar structure, but have a higher target training effect (aerTE), for example 3.5 or even 3.7 in case of experienced athletes. It is also possible to exclude warm-ups and cooldowns totally, if a more simple workout structure is preferred.

According to an embodiment the easiest Tempo label workout structure in the Cycling exercise mode may have a target aerTE 3.8 and contain a 10 min warm-up at *Warm-up & Recovery zone* and an interval exercise with 3-5 repetitions of 8-10 min at *Tempo zone* with 5 min recovery at *Warm-up & Recovery zone* between the repetitions. The number and the duration of the repeats may be optimize so that aerTE is 3.8 and Tempo label (label 3) target will be reached after the whole workout. Alternatively, the exercise part of the workout structure may contain 30-60 min exercise at *Tempo zone* and the duration of the exercise is optimized so that aerTE is 3.8 and Tempo label (label 3) target will be reached after the whole workout. The workout structure may further contain a 15 min cooldown at *Warm-up & Recovery zone.* The hardest Tempo label workout in the Cycling exercise mode may have a similar structure, but have a higher target training effect (aerTE), for example 4.2. As disclosed above, any planned workout structure, that is, any label and any structure including all exemplary workout structure embodiments described below, may also be optimized before providing it to the user by utilizing a simulation where it can be checked that planned workout allows user to achieve all targets (aerTE, anTE, label). This check may be performed in real time once the target label and target training effect values are known: In essence, simulative-optimization phase may be done in real time. Alternatively the workouts may also be pre-optimized. For example, after the target label and training effect (aerTE and anTE) have been determined then label, activity class and target training effect (aerTE and anTE) values may be provided as an input for a function where the highest activity class (10) is associated with highest amount of repeats, sets and repeat durations and lowest activate class (0) is associated with lowest amount of repeats, sets and repeat durations. The function may utilize for example a linear interpolation approach.

According to an embodiment the easiest Lactate Threshold (LT) label workout structure in the Cycling exercise mode may have a target aerTE 3.8 and contain a 15 min warm-up at *Base zone* and an interval exercise with 2-4 repetitions of 6-8 min at *LT & FTP zone* with 4 min recovery at *Warm-up & Recovery zone* between the repeats. Alternatively, the exercise part of the workout structure may contain an interval exercise with 1-2 repetitions of 15-20 min at *LT & FTP zone* with 5 min recovery at *Warm-up & Recovery zone* between the repeats. The number and the duration of the repeats may be optimized so that aerTE is 3.8 and LT label (label 4) target will be reached after the whole workout. The workout structure may further contain a 15 min cooldown at *Warm-up & Recovery zone.* The hardest LT label workout in the Cycling exercise mode may have a similar structure, but have a higher target training effect (aerTE), for example 4.2.

According to an embodiment the easiest VO2max label workout structure in the Cycling exercise mode may have a target aerTE 3.8 and contain a 15 min warm-up at *Base zone* and an interval exercise with 3-5 repetitions of 3-4 min at VO2max *zone* with 2 min recovery at *Warm-up & Recovery zone* between the repeats. Alternatively, the exercise part of the workout structure may contain an interval exercise with 5-10 repetitions of 2 min at *VO2max zone* with 1 min recovery at *Warm-up & Recovery zone* between the repeats. The number and the duration of the repeats may be optimized so that aerTE is 3.8 and VO2max label (label 5) target will be reached after the whole workout. The workout structure may further contain a 15 min cooldown at *Warm-up & Recovery zone.* The hardest VO2max label workout in the Cycling exercise mode may have a similar structure, but have a higher target training effect (aerTE), for example 4.2.

According to an embodiment the easiest Anaerobic Capacity label workout structure in the Cycling exercise mode may have a target anaerobic training effect (anTE) 3.0 and contain a 30 min warm-up at *Base zone* and an interval exercise with 2-3 sets of 4-5 repetitions of 40 sec at *Anaerobic Capacity zone* with 20 sec recovery between the repeats and 5 min recovery between the sets at *Warm-up & Recovery zone.* Alternatively, the exercise part of the workout structure may contain an interval exercise with 1-2 sets of 3-4 repetitions of 60 sec at *Anaerobic Capacity zone* with 60 sec recovery between the repeats and 5 min recovery between the sets at *Warm-up & Recovery zone.* Alternatively, the exercise part of the workout structure may contain an interval exercise with 4-8 repetitions of 90 sec at *Anaerobic Capacity zone* with 4 min recovery between the repeats at *Warm-up & Recovery zone.* Alternatively, the exercise part of the workout structure may contain an interval exercise with 2-3 sets of 6-8 repetitions of 20 sec at *Speed zone* with 10 sec recovery between the repeats and 10 min recovery between the sets at *Warm-up & Recovery zone.* The workout structure further contains a 15 min cooldown at *Warm-up & Recovery zone.* The hardest Anaerobic Capacity label workouts in the Cycling exercise mode may have a similar structure, but have a higher number of repeats in each set and higher target training effect (anTE and aerTE), for example 3.3.

According to an embodiment the easiest Speed label workout structure in the Cycling exercise mode may have a target anaerobic training effect (anTE) 2.5 and contain a 20 min warm-up at *Base zone* and an interval exercise with 2-3 sets of 4-5 repetitions of 10 sec at *Speed zone* ("all out") with 2 min recovery between the repeats and 5 min recovery between the sets at *Warm-up & Recovery zone.* Alternatively, the workout structure may contain a 15 min warm-up at *Base zone,* an interval exercise with 4-8 repetitions of 20 sec at *Speed zone* ("all out") with 5 min recovery between the at *Warm-up & Recovery zone.* The workout structure further contains a 15 min cooldown at *Warm-up & Recovery zone.* The hardest Speed label workouts in the Cycling exercise mode may have a similar structure, but have a higher number of repeats in each set and higher target training effect (anTE and aerTE), for example 2.8.

According to an embodiment the easiest Rest and Recovery label workout structure in the Running exercise mode may have a target training effect (aerTE) 1.5 and contain an exercise at *Warm-up & Recovery zone* until aerTE 1.0 is reached. The hardest Rest and Recovery label workout in the Running exercise mode may have a similar structure, but have a higher target training effect (aerTE), for example 2.2.

According to an embodiment the Rest and Recovery label workout structure for a cooldown exercise in the Running exercise mode may have a target training effect (aerTE) 1.5 and contain an exercise at *Base zone* until aerTE 1.0 is reached followed by exercise at *Warm-up & Recovery* zone until aerTE 1.5 is reached.

According to an embodiment the easiest Base label workout structure in the Running exercise mode may have a target training effect (aerTE) 2.5 and contain a 10 min warm-up at *Warm-up & Recovery zone* and an exercise at *Base zone* until aerTE 2.5 is reached. If the VO2max of the user is below a certain threshold value (for example 55 ml/kg/min), workout structure may be more simple and just contain an exercise at *Base zone* until aerTE 2.5 is reached as less fit individuals (users) may not be able to run at *Warm-up & Recovery zone* with a good running technique as the pace would be so slow. Naturally, it is possible to exclude warm-ups even from the users with higher VO2 Max, if a more simple structure is preferred. The hardest Base label workout in the Running exercise mode may have a similar structure, but have a higher target training effect (aerTE), for example 3.5, or even 3.7 considering the most experienced athletes. In addition, Base label (label 2) target should be reached after the whole workout.

According to an embodiment the easiest Tempo label workout structure in the Running exercise mode may have a target aerTE 3.8 and contain a 10 min warm-up at *Warm-up & Recovery zone* and an interval exercise with 3-5 repetitions of 8-10 min at *Tempo zone* with 3 min recovery at *Warm-up & Recovery zone* between the repetitions. The number and the duration of the repeats may be optimized so that aerTE is 3.8 and Tempo label (label 3) target will be reached after the whole workout. Alternatively, the exercise part of the workout structure may contain 20-50 min exercise at *Tempo zone* and the duration of the exercise is optimized so that aerTE is 3.8 and Tempo label (label 3) target will be reached after the whole workout. The workout structure further contains a 10 min cooldown at *Base zone.* The hardest Tempo label workout in the Running exercise mode may have a similar structure, but have a higher target training effect (aerTE), for example 4.2. In addition, Tempo label (label 3) target should be reached after the whole workout.

According to an embodiment the easiest Lactate Threshold (LT) label workout structure in the Running exercise mode may have a target aerTE 3.8 and contain a 10 min warm-up at *Base zone* and an interval exercise with 2-4 repetitions of 6-8 min at *LT & FTP zone* with 2 min recovery at *Warm-up & Recovery zone* between the repeats. Alternatively, the exercise part of the workout structure may contain an interval exercise with 1-2 repetitions of 15-20 min at *LT & FTP zone* with 5 min recovery at *Warm-up & Recovery zone* between the repeats. The number and the duration of the repeats may be optimized so that aerTE is 3.8 and LT label (label 4) target will be reached after the whole workout. Alternatively, the exercise part of the workout structure may contain 15-30 min exercise at *LT & FTP zone* and the duration of the exercise is optimized so that aerTE is 3.8 and LT label (label 4) target will be reached after the whole workout. The workout structure further contains a 10 min cooldown at *Base zone.* The hardest LT label workout in the Running exercise mode may have a similar structure, but have a higher target training effect (aerTE), for example 4.2. In addition, LT label (label 4) target should be reached after the whole workout.

According to an embodiment the easiest VO2max label workout structure in the Running exercise mode may have a target aerTE 3.8 and contain a 10 min warm-up at Base *zone* and an interval exercise with 4-5 repetitions of 3-4 min at *VO2max zone* with 2 min recovery at *Warm-up & Recovery zone* between the repeats. Alternatively, the exercise part of the workout structure may contain an interval exercise with 5-10 repetitions of 2 min at *VO2max zone* with 2 min recovery at *Warm-up & Recovery zone* between the repeats. The number and the duration of the repeats may be optimized so that aerTE is 3.8 and VO2max label (label 5) target will be reached after the whole workout. The workout structure further contains a 10 min cooldown at *Base zone.* The hardest VO2max label workout in the Running exercise mode may have a similar structure, but have a higher target training effect (aerTE), for example 4.2. In addition, VO2max label (label 5) target should be reached after the whole workout.

According to an embodiment the easiest Anaerobic Capacity label workout structure in the Running exercise mode may have a target anaerobic training effect (anTE) 3.0 and contain a 15 min warm-up at *Base zone* and an interval exercise with 2-3 sets of 4-5 repetitions of 40 sec or 200 meters at 105-115% vVO2max, where vVO2max is an estimated or measured speed corresponding to estimated or measured VO2 Max, respectively, with 3min recovery between the repeats at *Warm-up & Recovery zone.* Alternatively, the exercise part of the workout structure may contain an interval exercise with 5-10 repetitions of 40 sec or 200 meters at *110-115% vVO2max* with 60 sec recovery between the repeats and 5 min recovery between the sets at *Warm-up & Recovery zone.* Alternatively, the exercise part of the workout structure may contain an interval exercise with 5-10 repetitions of 60 sec or 400 meters at 100-105% vVO2max with 3 min recovery between the repeats at *Warm-up & Recovery zone.* The workout structure further contains a 10 min cooldown at Base *zone.* The hardest Anaerobic Capacity label workout in the Running exercise mode may have a similar structure but have a higher number of repeats in each set and higher target training effect (aerTE and anTE), for example 3.3.

According to an embodiment the easiest Speed label workout structure in the Running exercise mode may have a target anaerobic training effect (anTE) 2.5 and contain a 15 min warm-up at *Base zone* and an interval exercise with 1-2 sets of 4-5 repetitions of 10 sec or 50 meters at *130-150% vVO2max* ("all out / 95%") with 3 min recovery between the repeats and 5 min recovery between the sets at *Warm-up & Recovery zone.* Alternatively, the exercise part of the workout structure may contain an interval exercise with 2-3 sets of 4-5 repetitions of 15 sec or 100 meters at *130-150% vVO2max* ("all out / 95%")%") with 3 min recovery between the repeats and 5 min recovery between the sets at *Warm-up & Recovery zone.* The workout structure further contains a 10 min cooldown at *Base zone.* The hardest Speed label workout in the Running exercise mode may have a similar structure but have a higher number of repeats in each set and higher target training effect (aerTE and anTE), for example 2.8.

According to an embodiment the easiest Rest and Recovery label workout structure in the Generic exercise mode may have a target training effect (aerTE) 1.5 and contain an exercise at *Warm-up & Recovery zone* until aerTE 1.0 is reached. The hardest Rest and Recovery label workout in the Generic exercise mode may have a similar structure, but have a higher target training effect (aerTE), for example 2.2.

According to an embodiment the Rest and Recovery label workout structure for a cooldown exercise in the Generic exercise mode may have a target training effect (aerTE) 1.5 and contain an exercise at *Base zone* until aerTE 1.0 is reached followed by exercise at *Warm-up & Recovery* zone until aerTE 1.5 is reached.

According to an embodiment the easiest Base label workout structure in the Generic exercise mode may have a target training effect (aerTE) 2.5 and contain an exercise at *Base zone* until aerTE 2.5 is reached. The hardest Base label workout in the Generic exercise mode may have a similar structure, but have a higher target training effect (aerTE), for example 3.3. In addition, Base label (label 2) target should be reached after the whole workout.

According to an embodiment the easiest Tempo label workout structure in the Generic exercise mode may have a target aerTE 3.8 and contain a 10 min warm-up at *Base zone* and a 20-50 min exercise at *Tempo zone.* The duration of the exercise is optimized so that aerTE is 3.8 and Tempo label (label 3) target will be reached after the whole workout. The workout structure further contains a 10 min cooldown at *Base zone.* The hardest Tempo label workout in the Generic exercise mode may have a similar structure, but have a higher target training effect (aerTE), for example 4.2. In addition, Tempo label (label 3) target should be reached after the whole workout.

According to an embodiment the easiest Lactate Threshold (LT) label workout structure in the Generic exercise mode may have a target aerTE 3.8 and contain a 10 min warm-up at *Base zone* and a 15-30 min exercise at *LT & FTP zone.* The duration of the exercise is optimized so that aerTE is 3.8 and LT label (label 4) target will be reached after the whole workout. The workout structure further contains a 10 min cooldown at *Base zone.* The hardest LT label workout in the Generic exercise mode may have a similar structure, but a higher target training effect (aerTE), for example 4.2. In addition, LT label (label 4) target should be reached after the whole workout.

**NWR feedback phrases**

In addition to the workout structure, a feedback phrase is also provided to a user at each time the training history data is updated. The feedback phrase explains why the algorithm has chosen a certain recommendation. Examples of the phrase determination process are given in table 9.

Table 9. Calculated workout triggers and associated NWR and feedback phrases

| NWR Feedback number | workout phrase key words | Trigger | Example Feedback |
|---|---|---|---|
| 1 | RUNNING HISTORY REST | NWR-Label = "Rest" AND weekly allowed running distance is exceeded | "Your weekly running distance is pretty high. Today rest." |
| 2 | POOR SLEEP RUNNING HISTORY REST | NWR-Label = "Rest" AND Sleep score < 25 AND weekly allowed running distance is exceeded | "Your weekly mileage is pretty high and you had poor sleep last night. Better to rest today" |
| 3 | POOR SLEEP REST | NWR-Label = "Rest" AND Sleep score < 25 | "Poor sleep quality detected. Today rest" |
| 4 | POOR SLEEP RECOVERY | NWR-Label ="Rest or Active Recovery" AND Sleep score < 25 | "Poor sleep quality detected. Today rest or active recovery." |
| 5 | POOR SLEEP BASE | NWR-Label = "Base" AND 25 < = Sleep score < 40 | "Suboptimal sleep quality may slightly reduce your training readiness but you can still perform a base workout today." |
| 6 | HIGH RECOVERY TIME BASE | NWR-Label = "Base" AND Recovery time >=36 h | "Your recovery from prior training is not yet complete but status is still good enough in order to do a base workout." |
| 7 | OVERREACHING BASE | NWR-Label = "Base" AND Training Status = Overreaching AND all workout labels today ["until now"] AND yesterday AND day before yesterday are either "Recovery" or "Rest" | "Regardless of overreaching status it is better to do an easy base workout today rather than to rest many days in a row" |
| 8 | OVERREACHING REST | NWR-Label = "Rest" AND Training Status = Overreaching AND either Today OR yesterday OR day before yesterday contain a workout with a label "Base", "Tempo", "LT/FTP", "VO2max", "Anaerobic Capacity" or "Speed" | "Overreaching status-Today rest" |
| 9 | OVERREACHING RECOVERY | NWR-Label = "Rest or Active Recovery" AND TRAINING STATUS = Overreaching AND EITHER Today OR yesterday OR day before yesterday contain a workout with a label "Base" or higher | "Overreaching status - Keep main focus in rest or active recovery" |
| 10 | EASY WEEKLOAD REST | NWR-Label = "Rest" AND easy base workout (aerTE2.5 & anTE 0.0) today would cause exceeding WTL_target AND week cycle = Easy | "You are already close or above optimal load allocated for this easy week - Today rest" |
| 11 | MEDIUM WEEKLOAD REST | NWR-Label = "Rest" AND easy base workout (aerTE2.5 & anTE 0.0) today would cause exceeding WTL_target AND week cycle Medium | "You are already close or above optimal load allocated for this medium week - Today rest" |
| 12 | HARD WEEKLOAD REST | NWR-Label = "Rest" AND easy base workout (aerTE2.5 & anTE 0.0) today would cause exceeding WTL_target AND week cycle = Hard | "You are already close or above optimal load allocated for this hard week - Today rest" |

(continued)

| NWR Feedback number | workout phrase key words | Trigger | Example Feedback |
|---|---|---|---|
| 13 | EASY WEEKLOAD RECOVERY | NWR-Label = "Rest or Active recovery" AND easy base workout (aerTE2.5 & anTE 0.0) today would cause exceeding WTL_target AND week cycle Easy | "You are already close or above optimal load allocated for this easy week - Today " rest or active recovery |
| 14 | MEDIUM WEEKLOAD RECOVERY | NWR-Label = "Rest or Active recovery" AND easy base workout (aerTE2.5 & anTE 0.0) today would cause exceeding WTL_target AND week cycle Medium | "You are already close or above optimal load allocated for this medium week - Today rest or active recovery" |
| 15 | HARD WEEKLOAD RECOVERY | NWR-Label = "Rest or Active recovery" AND easy base workout (aerTE2.5 & anTE 0.0) today would cause exceeding WTL_target AND week cycle Hard | "You are already close or above optimal load allocated for this hard week - Today rest or active recovery" |
| 16 | EASY WEEKLOAD BASE | NWR-Label = "Base" AND easy base workout (aerTE2.5 & anTE 0.0) today would cause exceeding WTL_target AND week cycle = Easy AND today and yesterday is rest | "You are already close or above optimal load allocated for this easy week but you have been recovering yesterday and today. Today Base." |
| 17 | MEDIUM WEEKLOAD BASE | NWR-Label = "Base" AND easy base workout (aerTE2.5 & anTE 0.0) today would cause | "You are already close or above optimal load allocated for this medium week but you have been |
|  |  | exceeding WTL_target AND week cycle = Medium AND today and yesterday is rest | recovering yesterday and today. Today Base." |
| 18 | HARD WEEKLOAD BASE | NWR-Label = "Base" AND easy base workout (aerTE2.5 & anTE 0.0) today would cause exceeding WTL_target AND week cycle = Hard AND today and yesterday is rest | "You are already close or above optimal load allocated for this hard week but you have been recovering yesterday and today. Today Base." |
| 19 | TODAY LOAD REST | NWR-Label = "Rest" AND More than "load corresponding to aerTE 2.5" performed today in single or multiple workouts OR run distance today > 5 km OR bike distance today > 20 km | "Significant amount of activity performed for today - Rest for the remainder of today" |
| 20 | TODAY LOAD RECOVERY | NWR-Label = "Rest or Active Recovery" AND More than "load corresponding to aerTE 2.5" performed today in single or multiple workouts OR run distance today > 5 km OR bike distance today > 20 km | "Significant amount of activity performed for today - Rest or active recovery recommended for the remainder of today" |
| 21 | AEROBIC HIGH SHORTAGE | NWR-Label = "Tempo", "LT/FTP or "VO2max" AND Training Load Distribution feedback = "Aerobic high shortage" | "High aerobic workout needed for today to fix the shortage. This type of workout will be recommended a bit more frequently until balanced load focus is achieved" |
| 22 | ANAEROBIC CAPACITY | NWR-Label = "Anaerobic Capacity" | "Enough aerobic training in short-term history. Today it's a good time for an anaerobic workout" |

(continued)

| NWR Feedback number | workout phrase key words | Trigger | Example Feedback |
|---|---|---|---|
| 23 | ANAEROBIC SPEED | NWR-Label = "Speed" | "Enough aerobic training in short-term history. Today it's |
| | | | a good time for a speed workout" |
| 24 | AEROBIC LOW SHORTAGE BASE | NWR-label = "Base" AND Training Load Distribution Feedback phrase = "Aerobic Low Shortage", "Aerobic High Focus", "Anaerobic Focus", "Above targets" | "Today base workout in order to keep Training load Focus in better balance" |
| 25 | RUNNING HISTORY IS SHORTENED BASE | Recommended duration with actual training history is 30min or more shorter than one could might get with a perfect history | "Distance and duration of this (running) workout has been downgraded due to your limited running experience" |
| 26 | RUNNING HISTORY IS REPLACED BASE | NWR-label = "Base" AND last 8-day total running distance < 5 km AND 7 -day load > 60 units (recommended cycling workout is aerobic high or anaerobic) | "Some low intensity running is needed before it is sensible and safe to make any harder runs. Today base" |
| 27 | TRAINING HISTORY IS REPLACED BASE | NWR-label = "Base" AND last 8-day total running distance < 5 km AND load < 60 units | "Some low intensity training is needed before progressing to harder workouts. Today base" |
| 28 | RUNNING HISTORY IS DOWNGRADED HIGH AEROBIC | NWR-label = "Tempo", "LT/FTP" or "VO2max" BUT high intensity workout has been "eased" due to poor running history | "This high intensity running workout has been eased due to your sub-optimal running experience and it may thus not have optimal impact on your cardiorespiratory system. You could perform a bit harder workout if you select some other sport, such as cycling, with less impact forces" |
| 29 | RUNNING HISTORY IS DOWNGRADED ANAEROBIC | NWR-label = "Anaerobic Capacity" or "Speed" BUT workout has been "eased" due to poor running history | "This high intensity running workout has been eased due to your sub-optimal running experience and it may thus not have optimal impact on your muscle power and anaerobic |
| | | | performance. You could perform a bit harder workout if you select some other sport, such as cycling, with less impact forces" |
| 30 | LOW WTL SHORTENED RUN BASE | If 7-day load < 2.5 (optimal low limit) and "Base" workout's running distance limited to 15% of maximum weekly mileage | "Despite low load, this base is kept short due to a longer run performed recently" |
| 31 | LACTATE THRESHOLD | Else if NWR-label = "L T/FTP" | "Everything is progressing well. Today an LT [FTP] workout" |
| 32 | TEMPO | Else if NWR-label = "Tempo" | "Everything is progressing well. Today a Tempo workout" |

(continued)

| NWR Feedback number | workout phrase key words | Trigger | Example Feedback |
|---|---|---|---|
| 33 | VO2MAX | Else if NWR-label = "VO2max" | "Everything is progressing well. Today a VO2max workout" |
| 34 | BASE | Else if NWR-label = "Base" | "All balanced but still better to keep intensity low today to optimize the day level periodization. Today Base" |
| 35 | RECOVERY | Else if NWR-label = "Rest or active recovery" | "Everything is progressing well - Today Rest or Active recovery." |
| 36 | REST | Else if NWR-label = "Rest" | "Everything is progressing well - Today Rest" |
| 37 | POOR SLEEP LOAD REST | NWR-Label = "Rest" AND Sleep score < 25 AND easy base workout (aerTE2.5 & anTE 0.0) today would cause exceeding WTL_target | "Your weekly load is pretty high and you had poor sleep last night. Better to rest today" |
| 38 | MODERATE SLEEP LOAD BASE | NWR-Label = "Base" AND 25 < Sleep score < 40 AND easy base workout (aerTE2.5 & anTE 0.0) today would cause exceeding WTL_target | "You are close or above load allocated for this week and you had a suboptimal sleep last night. Better to do only easy base workout today." |
| 39 | RUNNING HISTORY RECOVERY | NWR-Label = "Rest or Active Recovery" AND personal weekly allowed running distance is exceeded | "Your weekly running distance is pretty high. Today rest or active recovery." |
| 40 | POOR SLEEP RUNNING HISTORY RECOVERY | NWR-Label = "Rest or Active Recovery" AND Sleep score < 25 AND personal weekly allowed running distance is exceeded | "Your weekly running distance is pretty high and you had poor sleep last night. Today rest or active recovery." |
| 41 | POOR SLEEP LOAD RECOVERY | NWR-Label = "Rest or Active Recovery" AND Sleep score < 25 AND AND easy base workout (aerTE2.5 & anTE 0.0) today would cause exceeding WTL_target | "Your weekly load is pretty high and you had poor sleep last night. Today rest or active recovery." |
| 42 | NO HISTORY BASE | NWR = 2 AND No history available (unboxing situation) | "We have no training information from you yet. Let's start with an easy base workout". |
| 43 | MODERATE SLEEP RUNNING HISTORY RECOVERY | NWR-label = "Rest or Active Recovery" AND 25 < sleep_score < 40 AND personal weekly allowed running distance exceeded | "Your weekly running distance is pretty high and you did not have optimal sleep last night either. Today rest or active recovery." |
| 44 | MODERATE SLEEP RUNNING HISTORY REST | NWR-label = "Rest" AND 25 < sleep_score < 40 AND weekly allowed running distance exceeded | "Your weekly running distance is pretty high and you did not have optimal sleep last night either. Today rest or active recovery." |
| 45 | HIGH RECOVERY TIME RUNNING HISTORY RECOVERY | NWR-label = "Rest or Active Recovery" AND recovery_time > = 36 h AND personal weekly allowed running distance exceeded | "Your recovery from prior training is not yet complete and your weekly running distance is high. Today rest or active recovery". |

(continued)

| NWR Feedback number | workout phrase key words | Trigger | Example Feedback |
|---|---|---|---|
| 46 | REST | NWR-label = "Rest" AND recovery_time > = 36 h AND personal weekly | "Your recovery from prior training is not yet complete and your weekly running |
| | | allowed running distance exceeded | distance is high. Today rest." |

**Additional rules related to selected workout and workout structure**

An NWR is redetermined in case at least one of the following is detected:

- a measured running distance during the training is too high compared to an estimated running tolerance of the user,
- a monthly training load is determined to be below a predetermined target;
- the total amount of the *aerobic low load* training is determined to be too low compared to the total amount of the training load or compared to the activity class of the user;
- the total amount of the *aerobic high load* training is determined to be too high compared to the total amount of the training load or compared to the activity class of the user;
- the total amount of the *aerobic high load* training is determined to be too low compared to the total amount of the training load or compared to the activity class of the user;
- the total amount of the *anaerobic load* training is determined to be too high compared to the total amount of the training load or compared the activity class of the user;
- proportion of the aerobic high intensity training is determined to be too low during a training cycle of past 4-14 days;
- proportion of the *anaerobic load* training is determined to be too high compared to the total amount of the training load or compared to the activity class of the user;
- proportion of the *anaerobic load* is determined to be too low compared to the total amount of the training load or compared to the activity class of the user;
- the training load is determined to be too high compared to the activity class of the user.

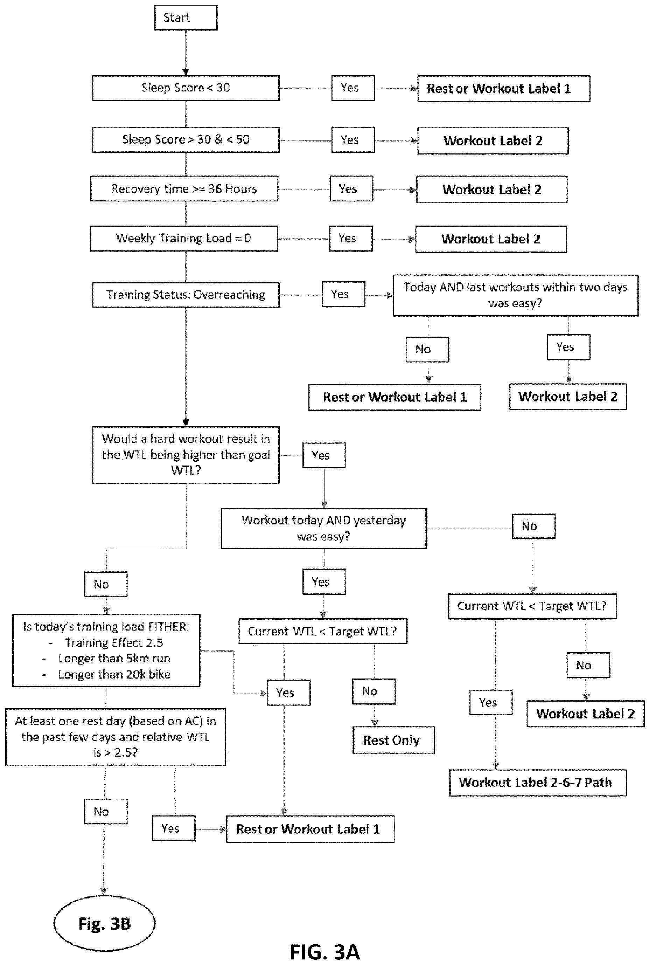

In the previous too high corresponds to a situation, wherein the NWR as originally determined, before redetermining, would cause overreaching or exceeding the compared limit. In the previous too low corresponds to a situation, wherein the NWR as originally determined, before redetermining, would cause underreaching the compared limit.
In order to avoid overtraining and overuse injuries the following additional rules may be included when defining the workout structure. The following exceptions are not shown in the flowchart of Figures 3A-D:

1. Running distance (in running and generic mode) is not allowed to exceed following limits:

- Considering a planned Label 2 workout - if accumulated running distance during the past seven days (from day 0 to day -6) is greater than 95% (easy), 120% (medium) or 135% (hard) of running tolerance where running tolerance is the maximum weekly average of past 5-10 weeks, then start prescribe REST (or label 1) and label 2 + aerTE 2.5 on successive days

    - If there is no information on mileage or weekly distance is less than 25 km, then use 25 km as a "guess" for these weeks.
    - Any "tolerance" higher than 100 km/week will be considered as 100 km.

- In a recommended label 2 workout, the maximum distance allowed for a single run is 25% - 30% (25% if weekly maximum is 60 km or more, 30% if weekly maximum is 40 km or less, with a sliding% in between 40-60 km) of highest mileage allowed for a single week (varies between easy (95%), medium (120%) and hard (135%) weeks).
- Sum of two longest runs within last 5 days (from day 0 to day -4) is not allowed to exceed 45% of maximum weekly mileage. However, even if they would exceed, "the latter" run is never shortened below 15% of the maximum weekly mileage. This shortening is not applied to Label 3-7 workouts.
- If two successive days seem to exceed 40% of maximum allowed weekly mileage, next workout is forced to Label 2, aerTE 2.5 workout.

2. In running AND cycling, Label 3-7 workouts may be eased or totally replaced with label 2 workouts if the running distance during last 28 day is 20 km or less and AC is 4 or below and

- In running mode - if total running distance during days last 7 days is less than 5 km, OR if weekly load < 60 units, then replace label 3-7 with a label 2 workout (including WTL & possible other running history limitations)
- In cycling mode - if weekly load < 60 units, then replace label 3-7 with a label 2 workout (including WTL & possible other running history limitations)

3. In order to add variation to the weekly recommendations, the following additional logic may be applied to cycling and running:

- Considering a planned Label 2 workout - If the last week (from day 0 to day -6) already comprises 3 or more days with total load corresponding aerTE >= 3.3, then available aerTE range is between 2.5 and 3.0
- Considering any planned workout - If yesterday (day -1) already comprises one label 2 workout having aerTE >= 3.3 OR two or more label 1-2 workout(s) with total load corresponding to aerTE >= 4.0, then available aerTE range for today is between 2.5 and 3.0 and label 2 is forced.

4. In order to avoid accidentally having a workout recommended as label 1 becoming a label 2 workout (thereby becoming a higher training load workout), label 1 workouts may have a workout duration limit, based on activity class. For example, a label 1 duration may substantially linearly increase from 20 min to 40 min with the AC increasing from 4 to 10. Label 1 duration 20 may correspond to the AC 4 and label 1 duration 40 may correspond to the AC 10.

In cycling this means that label 1 workouts are not provided at all to the user's having AC of 4-6.

Conversely, modifications may also be made in order to avoid unreasonably short/easy workout recommendations:

1. In cycling program, if recommended label 1 duration is shorter than 30 min that day may be forced into rest.
2. In training goal "maintain", a recommendation to rest may be provided instead of showing a recommendation to label 1 workouts.
3. if running workout target calculated for the user is shorter than 3 km, then recommendation may be set to 3 km and aerTE amended accordingly.
4. If minimum speed target calculated for the user for a workout is lower than 4 km/h then value may be amended to 4 km/h.

- If - after these amendments - target speed range becomes "narrower" than 1 km/h, also the maximum speed may be amended in a way that at least 1 km/h "width" is achieved.

5. If minimum power target calculated for the user for a workout corresponds to a MET level lower than 4 MET, then power may be amended to correspond 4 MET.

- Nevertheless, never prescribe lower intensity than 20 W.
- If - after these amendments - target power range becomes more narrow than 20 W, then also the maximum power may be amended in a way that at least 20 W "width" is achieved.

## Additional exceptions to training goal considering "Maintain"

In maintain

- Activity class (AC) may be determined only using VO2max
- If VO2max based AC is 4 or 5, AC may be increased based on monthly load until AC reaches AC 6 (target for any user is a active/healthy lifestyle). For example, if workout label 2-7 is achieved every day on days-1 to -(AC/2) AND Relative WTL >= 2.5 then a rest or label 1 recommendation is provided
- Label 2 workouts in maintain mode are always limited to aerTE values of 2.5 to 3.0

## Detection and determination of weekly training load target and weekly load rhythm

Weekly training load target may be selected in a way that the activity class of the user increases a given amount in a given time. For example, training load target in the improve mode may be set to increase 1 activity class per each month. In an exemplary embodiment this may be achieved by keeping the average relative weekly training load at the level of 3.5 (current activity class would be maintained, if load level is 3.0) on a monthly basis which may also correspond to a relative monthly load level of 3.5, which may be the required monthly load (relative monthly 3.0), to achieve and maintain the next higher activity class. I.e. training load may be kept continuously at 3.5 level which always "guarantees" achieving also the required monthly training load considering the one level higher activity class. Figure 10 is exemplary of this concept. For example, if a user has an activity class of 7, the weekly and monthly (4 week) training load target corresponding to the relative value of 3.5 are approximately 260 and 1040 training load units, respectively. As one can see from the figure, the approximately 260 units (or 1040 units / 4 weeks) corresponds to a relative weekly training load value of 3.0 for an activity class of 7.5. Accordingly, when the user follows next workout recommendations which keep the weekly load level at 260 units per week (1040 units per month), the user will reach the required level considering achieving 3.0 weekly load and 3.0 monthly load. This will lead to updating his/her activity class 7.5 even though his or her VO2Max had not improved.

Naturally, it is also possible to achieve the average monthly training load level of 3.5 by variating the weekly training load targets, for example, by following a rhythm of easy, medium and hard. This kind of exemplary embodiment is provided below:

Training load is intended to vary on a weekly basis in order to speed up fitness development. In an embodiment, variability may be added to the training program via modifying the target weekly training load index (tWTL) on a weekly-basis. The variation in weekly training load index may occur for example following index variation 2.5, 3.5, 4.2, 2.5, 3.5, 4.2 etc. Where each index value from 2 to 5 is scaled individually based on fitness level and/or training tolerance measured from prior training. The alignment between activity class and tWTL may be, for example, as presented in Figure 10.

An aim may be to follow a 3-week cycle (if the training program is followed exactly) as follows:

1. Easy week: tWTL_easy = 2.5 in maintain and improve programs and 3.0 in the improve fast program and whenever the activity class is at least 8.0.
2. Medium week: tWTL_medium = 3.5 in maintain and improve programs and 3.9 in improve fast program.
3. Hard week: tWTL_hard = 4.2 in maintain and improve program and 4.3 in improve fast program.

In this embodiment, the term "week" means a calendar week, generally from Monday to Sunday. The rhythm detection is based on the realized weekly training load index values on the two previous calendar weeks (WTL_week0, WTL_week1). The weekly training load (WTL) rhythm may be indexed and target weekly training loads (tWTLs) may be weighted in order to achieve variation in training weeks, for example between easy, medium and hard training weeks. The weights may be selected such that they describe thresholds between easy, medium and hard workouts. The weights may be selected in the range of 20-45 and 80-55, correspondingly. For example 0.35 and 0.65 as follows:

$$WTL\_0 = 0.35 * tWTL\_easy + 0.65 * tWTL\_moderate$$

$$WTL\_1 = 0.65 * tWTL\_moderate + 0.35 * tWTL\_hard$$

$$WTL\_2 = 0.35 * tWTL\_moderate + 0.65 * tWTL\_hard$$

$$WTL\_3 = 1.10 * tWTL\_hard$$

When choosing the type of week to be followed, an easy week is chosen if any of the following five conditions hold:

- If any of the current training load categories (*aerobic low, aerobic high,* or *anaerobic*) are above their target,
- There were at least five rest days on both the previous and second to previous calendar week; and in addition, the total number of individual workouts during the two previous calendar weeks is less than three,
- (WTL_week0 > WTL_2) and (WTL_week1 > WTL_2),
- (WTL_week0 > WTL_1) and (WTL_week1 > WTL_0) and (WTL_week0 > WTL_week1),
- (WTL_week0 > WTL_3).

A medium week is chosen if all of the following four conditions hold:

- an easy week was not chosen,

- (WTL_week0 < WTL_2),
- (WTL_week1 > WTL_1),
- (WTL_week0 < WTL_week1).

A hard week is chosen if all of the following conditions hold:

- an easy week was not chosen,
- a medium week was not chosen,
- (WTL_week0 > WTL_0),
- (WTL_week1 < WTL_0).

Finally, if none of the three options (easy, medium, hard week) was chosen at this point, a fallback option is the following:

- Calculate a "reference Monday" as the starting point of the 3-week cycle. The "reference Monday" may be calculated based on user's average training history up to 365-730 days in history. Since the cycle is three weeks long, there are three options for the reference starting point. The reference starting point is calculated using the age, gender, height and weight of the user. The goal is to divide the population evenly into three buckets, each corresponding to a different starting point for the 3-week cycle while keeping the reference starting point of a single user relatively constant.
- Calculate the current phase (i.e., is it an easy, medium or hard week) based on the assumption that the week starting on the aforementioned "reference Monday" was an easy week.

In the beginning of the training history (i.e., right after unboxing) or after a long break in exercising, the first workout might not have been carried out on a Monday. In such case, a separate 3-week cycle (which might not necessarily follow calendar weeks, i.e., start on Monday) may be followed during the first 35 days (5 weeks) after unboxing or a long break. Now, WTL_week0 corresponds to the realized weekly training load index at the end of the previous week (which may not be a calendar week).
Now, an easy week may be chosen if any of the following conditions hold:

- Any of the "above targets" appears today,
- It is an easy week according to the cycle starting on the date of the first exercise,
- (WTL_week0 > WTL_3).

A medium week is now chosen if all of the following conditions hold:

- An easy week was not chosen,
- It is a medium week according to the cycle starting on the date of the first exercise.

Finally, a hard week is now chosen if all of the following conditions hold:

- An easy week was not chosen,
- A medium week was not chosen,
- It is a hard week according to the cycle starting on the date of the first exercise.

The weekly rhythm detection logic may be implemented in a function or an application module. Based on the simulations, where the recommended workouts are executed exactly as prescribed, the cycle detection logic enables providing variability in the WTL target level.

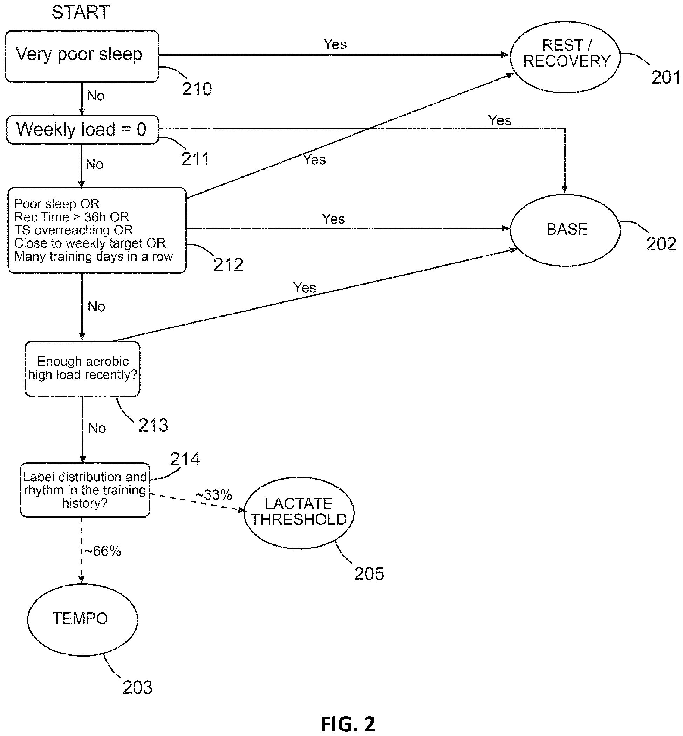

Figure 2 shows an exemplary simplified flowchart for providing a generic next workout recommendation according to an embodiment that is not specific to either running or cycling. An amount and quality of sleep is evaluated at phase 211. In case sleep quality is very poor, rest/recovery 201 is recommended. Otherwise evaluation proceeds to the next phase 212. At this phase 212 quality of sleep, WTL, recovery time, training status, weekly training load at present and successive training days are evaluated. In case predetermined threshold values are reached, as discussed with the Figure 1, Base training 202 or rest/recovery 201 is recommended. Otherwise evaluation proceeds to the next phase 213.
At phase 213 it is evaluated whether recent training includes enough *aerobic high load.* If this is the case, Base training 202 is recommended. This ensures that aerobic high trainings are not recommended too often, or successively. Instead an aerobic high detection is always followed by recommendation of Base training 202. In this embodiment of a "generic" mode, as in not specific to either cycling or running, anaerobic workouts are never recommended.

If the phase 213 is determined negative, label distribution and rhythm in the training history is evaluated at phase 214. Based on such, either Tempo 207 or LT training 205 is recommended. Similarly, in this "generic" mode embodiment, VO2max workouts are not recommended, and the recommended label distributions are adjusted to be 66% for tempo and 33% to lactate threshold.

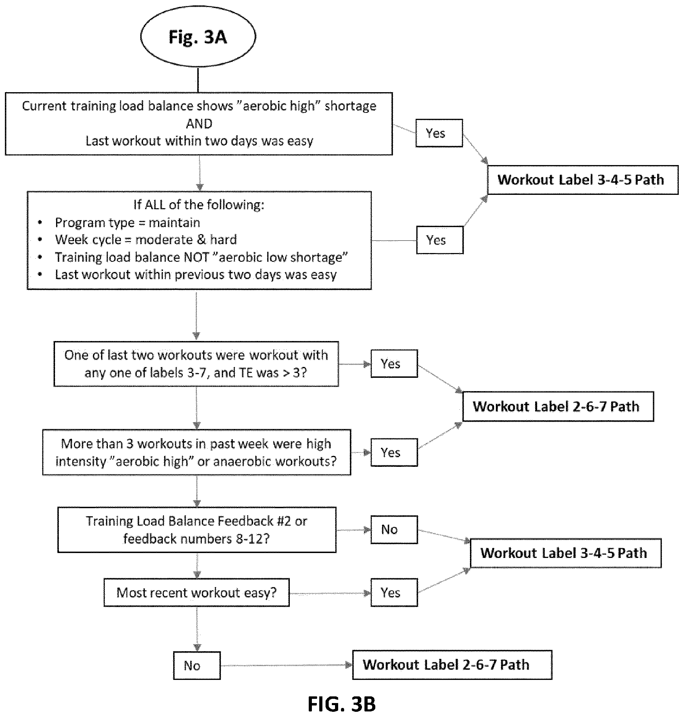

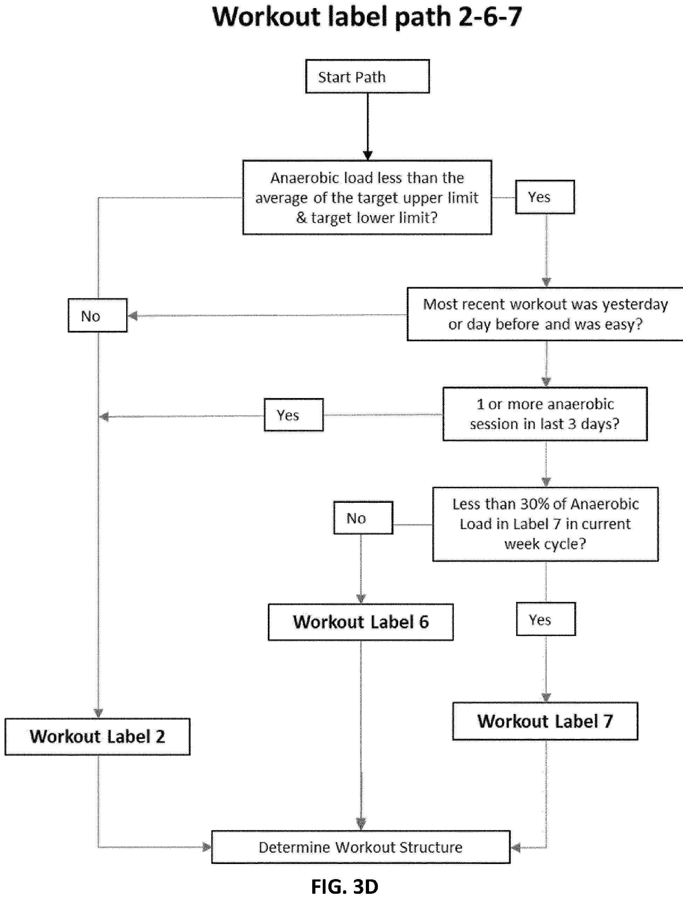

Figure 3A shows an exemplary flowchart for providing a next workout recommendation according to an embodiment. In the Figure 3A the initial determination for the next workout recommendation starts with the determination of sleep score. If sleep score is low, for example 25, workout label 1 is recommended. If the sleep score is between 25 and 40, workout label 2 is recommended. If recovery time from previous exercise is less than 36 hours, workout label 2 is recommended. Similarly, if weekly training load, WTL, is zero, workout label 2 is recommended. If training status can be described as "overreaching" and there has been a rest day or a workout with label 1 (in the exemplary Figures 3A-3D, referred to as an "easy" workout) within last two days workout label 2 is recommended, otherwise workout label 1 is recommended. If a workout with a aerTE value greater than 2,5 (in the exemplary Figures 3A-3D, referred to as a "hard" workout) would result in WTL being higher that the WTL target and there has been a rest day or a label 1 workout today and yesterday, NWR is rest if the current WTL is not below target WTL, and rest or workout label 1 if the current WTL is below target WTL. If a hard workout would result in WTL being higher that the WTL target and there has not been an easy workout within two days, NWR is workout label 2 if the current WTL is not below target WTL, and workout label from 2-6-7 path described in Figure 3D if the current WTL is below target WTL. If a hard workout would not result in WTL being higher that the WTL target and today's training load has training effect above 2,5, longer than 5 km run, longer than 20 km bicycle exercise or there have been multiple hard training days, rest or workout label 1 is recommended. If a hard workout would not result in WTL being higher that the WTL target and today's training load does not include training effect above 2,5, longer than 5 km run, longer than 20 km bicycle exercise and there has been at least one rest or easy day in the past few days, recommendations are determined as described in Figure 3B. In this embodiment, the "past few days" may be defined by the activity class of the user, so that users with a higher activity class may be able to perform more consecutive days of exercise without a rest or easy workout. The number of days for the rest day window may be calculated by dividing the activity class value by 2 and then adding 1. For a user with an activity class of 6, this would mean it would check for the rest days within the past 4 days.

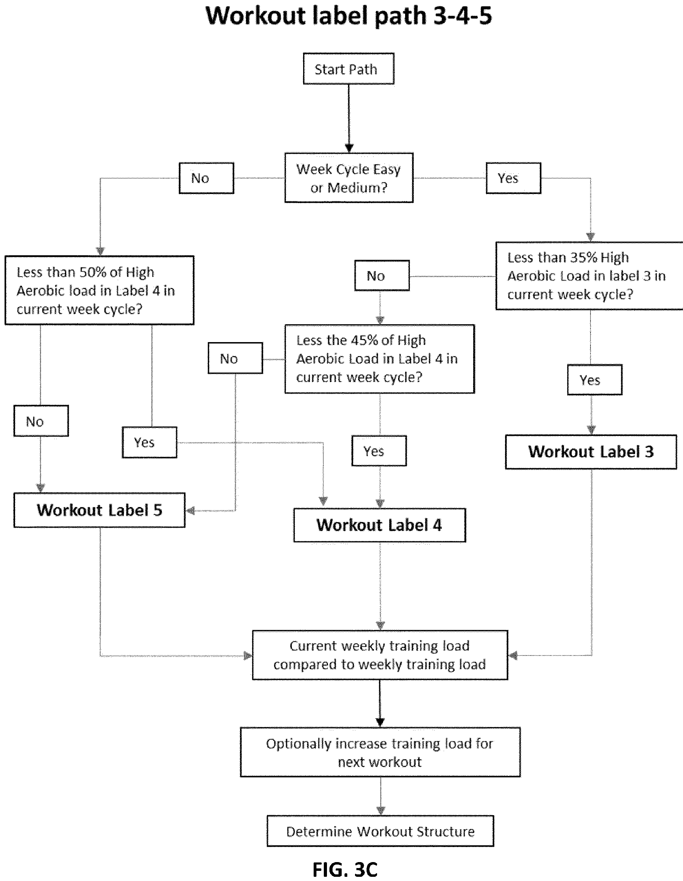

Figure 3B shows an exemplary flowchart for providing a next workout recommendation according to an embodiment. Figure 3B continues from the NWR described in Figure 3A, where there was no hard workout resulting in WTL being higher that the WTL target and today's training load did not include training effect (aerTE) above 2,5, longer than 5 km run, longer than 20 km bicycle exercise and there had not been multiple hard training days. Thereafter, if the training load balance does not show an "aerobic load" shortage and the last workout within two days was easy, workout label from the 3-4-5 path described in Figure 3C is recommended. Thereafter, if the program goal is *maintain,* training cycle is medium or hard, training load balance (TLB, see also table 6) is not *aerobic low* shortage or previous workout within two days has been easy, workout label from the 3-4-5 path described in Figure 3C is recommended. Thereafter, if primary label in one of previous two workouts has been any of the workout labels 3-7 and the training effect value (aerTE or antTE) is greater than 3.0, workout label from the 2-6-7 path described in Figure 3D is recommended. Thereafter, if more than 3 workouts in past week have been any of the workout labels 3-7 and the training effect value (aerTE or anTE) is greater than 3.0, workout label from the 2-6-7 path described in Figure 3D is recommended. Thereafter, if training load balance feedback, as described in table 4 is not number 2 or feedback numbers 8-12, workout label from the 3-4-5 path described in Figure 3C is recommended. Thereafter, if the most recent workout was easy, workout label from the 3-4-5 path described in Figure 3C is recommended else workout label from the 2-6-7 path described in Figure 3D is recommended.

Figure 3C shows an exemplary flowchart for providing a next workout recommendation according to an embodiment. Figure 3C describes the 3-4-5 pathway of the NWR and requires predetermination of the NWR phase from Figures 3A and 3B. If the week cycle is easy or medium and less than 35% of the *aerobic high load* training has label 3, workout label 3 is recommended. If the week cycle is easy or medium and 35% or more of the *aerobic high load* training has label 3 and less than 45% of the *aerobic high load* training has label 4, workout label 4 is recommended. On the other hand, if the week cycle is easy or medium and 35% or more of the *aerobic high load* training has label 3, but 45% or more of the *aerobic high load* training has label 4, workout label 5 is recommended. Similarly, if the week cycle is not easy or medium and less than 50% of the *aerobic high load* training has label 4, workout label 4 is recommended, otherwise, if it is not less than 50%, workout label 5 is recommended. After determination of the workout label, current training load is compared to weekly training load. If the default next workout recommendation does not exceed the target weekly training load, a slightly harder workout still belonging to the same workout label may be presented. If the default next workout recommendation does not exceed the target weekly training load, the target training load may be increased by selecting the lowest of either a higher pre-defined target training effect training load value, such as TE4.2 or the actual remaining difference between the weekly training load target and the current weekly training load target. After determination of the workout label, workout structure is determined.

Figure 3D shows an exemplary flowchart for providing a next workout recommendation according to an embodiment. Figure 3D describes the 2-6-7 pathway of the NWR and requires predetermination of the NWR phase from Figures 3A and 3B. If the *anaerobic load* is less than the average between the weekly training load target upper limit and target lower limit and the previous workout within last two days has been easy and there has not been an anaerobic workout in last 3 days, workout label 7 is recommended if less than 30% of the *anaerobic load* has label 7 in the current week cycle and workout label 6 is recommended if 30% or more of the *anaerobic load* has label 7 in the current week cycle. If the *anaerobic load* is more than the average between the weekly training load target upper limit or previous workout within last two days has not been easy or there has been an anaerobic workout in last 3 days, workout label 2 is recommended. After determination of the workout label, workout structure is determined.

Figure 11 is a non-limiting block-diagram illustrating an exemplary apparatus. Figure 11 illustrates an apparatus for providing an adaptive training coach. Figure 11 shows a simplified block diagram of the apparatus. The apparatus comprises a module APPL, at least one processor μP, at least one memory MEM and a user interface module UI. In addition, input for the apparatus may be provided from a heart rate sensor associated with a controller CTRL1. Optionally, a motion sensor and a second controller, CTRL2, may be employed for the apparatus. The at least one memory MEM is configured to store or record information and executable instructions.

The module APPL comprises modules, for example executable instructions, configured to determine next workout recommendations, and variables related thereto. The module APPL may include, for example, executable instructions configured to access and/or receive user background information; executable instructions configured to determine a present activity class of a user; executable instructions configured to calculate/compare variables; executable instructions configured to determine and/or update a monthly training load; executable instructions configured to determine and/or update a weekly training load; executable instructions configured to determine an aerobic and/or an anaerobic training effect and - load of the workouts; executable instructions configured to determine a training goal of a user; executable instructions configured to provide a next workout out recommendation (NWR), executable instructions configured to access and/or process and/or add training history data; executable instructions configured to determine a recovery state of a user; executable instructions configured to determine a training status of a user; executable instructions configured to update a training load distribution; , executable instructions configured to determine primary and secondary labels; executable instructions configured to determine a training load target for a workout; and/or executable instructions configured to determine a sleep score, for example as illustrated in Figures 1-2, 3A-D. The apparatus may comprise means for implementing flow charts of Figures 1-2 and 3A-D, as described in the previous.

A user interface UI is configured to receive information inputted by a user and to present information. The user interface UI may be used to receive inputted information, like user background information and/or to present information, like presenting training plan information or a next workout recommendation(s) to a user.

One or more different data libraries may be used to calculate different time ranges of the training load distribution, for example the cumulative training history of a user and the real-time anTE and aerTE values of the user. Historical training load distribution may be calculated using a different software library (for example a Training History Analysis - THA library) than the library that calculates aerTE and anTE values, feedback phrases and workout labels for a specific workout. This may help in saving computational power as these calculation processes need not be performed at regular intervals (for example 5 sec intervals) but instead, may be calculated only after a new workout or in the beginning of a new day.

In addition to load sums for each intensity category, a library may also calculate load target ranges for each intensity category, as shown in Figure 9.

Exemplary cumulative load targets per each intensity category shown in Figure 9 may be determined, for example, using an athlete database wherein the limits are based on averages and standard deviations observed in subgroup of athletes whose fitness levels have been developing faster than the group average. The limits may also take into account user's activity class and are described in more detail below.

The feedback presented to a user may be presented on a screen of an apparatus. This feedback may be, for example, based on the realized training load of the training history as compared to the training load target values for each of the aforementioned categories. In addition to the graphical information shown, additional feedback phrases and sentences (as shown in tables 8 and 9) may be presented to the user on the selected apparatus.

**Claims**

1. A method for providing a next workout recommendation (NWR) comprising,

    - calculating an activity class for a user based on training history data and/or present fitness level of the user;
    - determining a total training load target based on the activity class and a training goal; wherein the training goal relates to maintaining or changing the fitness level of the user;
    - determining labels for performed workouts from the training history data, wherein a label describes a training effect of a workout, based on one or more of the measured intensities during workouts, calculated aerobic training effect and calculated anaerobic training effect; wherein the labels are further divided into intensity categories of *aerobic low load, aerobic high load and anaerobic load,* wherein each of the intensity categories comprises one or more labels,

- detecting a training load distribution from the training history data, wherein the training load distribution comprises a cumulative training load sum for the intensity categories of *aerobic low load, aerobic high load* and *anaerobic load,*

- calculating a weekly training load target based on the activity class and the training goal,

- calculating a weekly training load based on the training history data,

- determining the intensity category for the NWR based on recovery time from the previous training, a weekly training load compared to the weekly training load target and the training load distribution, and

- determining the NWR based on the labels of the previous workouts, determined weekly training load and the training load distribution, wherein the NWR comprises at least an aerobic training effect target, an anaerobic training effect target and/or the selected label.

2. The method according to claim 1, comprising determining a training load target for each intensity category based on the training history data.

3. The method according to any of claims 1-2, wherein each intensity category comprises at least 2 labels; and/or wherein the *low aerobic load* intensity category comprises the labels of Recovery and aerobic Base; wherein the *high aerobic load* category comprises the labels of Tempo, Lactate Threshold and VO2max; wherein the *anaerobic load* category comprises the labels of Anaerobic Capacity and Speed.

4. The method according to any of claims 1-3, wherein the workout comprises at least a primary label describing a primary training effect of the workout, and optionally additionally a secondary label describing a secondary training effect of the workout.

5. The method according to any of claims 1-4, wherein the primary label is accumulating the load in the intensity category it belongs to and altering the training load distribution; and if existing, the optional additional secondary label, is accumulating the load in the intensity category it belongs to and altering the training load distribution.

6. The method according to any of claims 1-5, comprising taking into account at least the primary labels in the training history, when selecting the label for the NWR.

7. The method according to any of claims 5-6, wherein in case the primary label is from the intensity category of *aerobic low load* or *aerobic high load,* then the optional additional secondary label, is in the intensity category of *anaerobic load;* and in case the primary label is in the intensity category of *anaerobic load,* then the optional additional secondary label, is in the intensity category of *aerobic low* or *aerobic high load.*

8. The method according to any of claims 1-7, comprising determining the intensity category for the label for the NWR based on a sleep score, wherein the sleep score is at least partly based on sleep duration and/or sleep quality.

9. The method according to any of claims 1-8, comprising determining the weekly training load based on a training cycle, wherein the training cycle comprises 4-14 days, and/or the training cycle comprises one of: easy, medium and hard.

10. The method according to any of claims 1-9, comprising determining a training status based on the weekly training load, and a change in the weekly training load with respect to the previous workouts, and optionally taking into account the fitness level and the recovery state of a user.

11. The method according to any of claims 1-10, wherein the training history data comprises training load sums and activity class based training load target ranges for each intensity category.

12. The method according to any of the claims 1-11, comprising defining a label for the NWR based on at least one of: the primary labels of the current and the previous day workouts, recovery time from the previous training, the training load distribution, the labels in the training history, a current measured load of the training cycle compared to the target training load of the training cycle.

**13.** The method according to any of claims 1-12, comprising redetermining NWR, in case at least one of the following is detected:

- a measured running distance during the training cycle is too high compared to an estimated running tolerance of the user,
- a monthly training load is determined to be below a predetermined target;
- the total amount of the *aerobic low load* training is determined to be too low compared to the total amount of training load or compared to the activity class of the user;
- the total amount of the *aerobic high load* training is determined to be too high compared to the total amount of the training load or compared to the activity class of the user;
- the total amount of the *aerobic high load* training is determined to be too low compared to the total amount of the training load or compared to the activity class of the user;
- the total amount of the *anaerobic load* training is determined to be too high compared to the total amount of the training load or the activity class of the user;
- proportion of the aerobic high intensity training is determined to be too low during a training cycle of past 4-14 days;
- proportion of the *anaerobic load* is determined to be too high compared to the total amount of the training load or compared to the activity class of the user;
- proportion of the *anaerobic load* is determined to be too low compared to the total amount of the training load or compared to the activity class of the user;
- the training load is determined to be too high compared to the activity class of the user;

wherein too high corresponds to a situation, wherein the NWR as originally determined, before redetermining, causes exceeding the compared limit;

wherein too low corresponds to a situation, wherein the NWR as originally determined, before redetermining, causes underreaching the compared limit.

**14.** The method according to any of claims 1-13, wherein the labels are determined in real-time during the workouts.

**15.** The method according to any of claims 1-14, wherein defining the NWR includes estimation of a training readiness, and/or of an injury risk.

**16.** The method according to any of claims 1-15, wherein the NWR comprises a workout structure, which includes at least one of the following:

- the label
- a workout phrase associated to the label,
- a duration of the NWR,
- a running or cycling distance,
- a training effect,
- an anaerobic training effect,
- a number of interval repeats,
- a number of set repeats,
- a workout profile for a warm-up phase,
- a workout profile for a work phase,
- a workout profile for an interval rest,
- a workout profile for a set rest,
- a workout profile for a cooldown phase.

**17.** The method according to claim 16, wherein the label and the workout structure for the NWR are exercise mode specific such that exercise mode specific information of the training history data has effect on the label and the workout structure.

**18.** Apparatus for providing a next workout recommendation, NWR, comprising means for implementing the method according to any of claims 1-17.

**19.** A computer product for providing a next workout recommendation, NWR, comprising executable instructions, which when executed by a processor are arranged to implement the method according to any of claims 1-17.

FIG. 1

START

Very poor sleep ─────── Yes ──────→ ( REST / RECOVERY )  201

210

│ No
↓

Weekly load = 0 ─────── Yes ──────→

211

│ No
↓

Poor sleep OR
Rec Time > 36h OR ─── Yes ──→ ( BASE )  202
TS overreaching OR
Close to weekly target OR
Many training days in a row

212

│ No
↓

Enough aerobic
high load recently? ─── Yes ──→

213

│ No
↓

214
Label distribution and
rhythm in the training ┈ ~33% ┈→ ( LACTATE THRESHOLD )
history?                                              205

│ ~66%
↓

( TEMPO )

203

**FIG. 2**

Start

Sleep Score < 30 → Yes → **Rest or Workout Label 1**

Sleep Score > 30 & < 50 → Yes → **Workout Label 2**

Recovery time >= 36 Hours → Yes → **Workout Label 2**

Weekly Training Load = 0 → Yes → **Workout Label 2**

Training Status: Overreaching → Yes → Today AND last workouts within two days was easy?

No → **Rest or Workout Label 1**

Yes → **Workout Label 2**

Would a hard workout result in the WTL being higher than goal WTL? → Yes

Workout today AND yesterday was easy? → No

No

Is today's training load EITHER:
- Training Effect 2.5
- Longer than 5km run
- Longer than 20k bike

At least one rest day (based on AC) in the past few days and relative WTL is > 2.5?

Yes → Current WTL < Target WTL?

Current WTL < Target WTL?

No → **Workout Label 2**

Yes → **Workout Label 2-6-7 Path**

Yes → **Rest or Workout Label 1**

No → **Rest Only**

No

Yes → **Rest or Workout Label 1**

Fig. 3B

**FIG. 3A**

44

$$\boxed{\textbf{Fig. 3A}}$$

| Current training load balance shows "aerobic high" shortage AND Last workout within two days was easy | → | Yes |
|---|---|---|

→ **Workout Label 3-4-5 Path**

| If ALL of the following:<br>• Program type = maintain<br>• Week cycle = moderate & hard<br>• Training load balance NOT "aerobic low shortage"<br>• Last workout within previous two days was easy | → | Yes |
|---|---|---|

| One of last two workouts were workout with any one of labels 3-7, and TE was > 3? | → | Yes |
|---|---|---|

**Workout Label 2-6-7 Path**

| More than 3 workouts in past week were high intensity "aerobic high" or anaerobic workouts? | → | Yes |
|---|---|---|

| Training Load Balance Feedback #2 or feedback numbers 8-12? | → | No |
|---|---|---|

**Workout Label 3-4-5 Path**

| Most recent workout easy? | → | Yes |
|---|---|---|

| No | → | **Workout Label 2-6-7 Path** |
|---|---|---|

## FIG. 3B

# Workout label path 3-4-5

```
                                    ┌──────────────┐
                                    │  Start Path  │
                                    └──────┬───────┘
                                           │
                                           ▼
         ┌──────┐              ┌──────────────────────┐              ┌──────┐
         │  No  │◄─────────────│   Week Cycle Easy     │─────────────►│ Yes  │
         └──┬───┘              │      or Medium?       │              └──┬───┘
            │                  └──────────────────────┘                 │
            │                                                           │
            ▼                          ┌──────┐         ┌────────────────────────────┐
 ┌─────────────────────┐               │  No  │────────►│  Less than 35% High         │
 │ Less than 50% of High│              └──┬───┘         │  Aerobic Load in label 3 in │
 │ Aerobic load in Label 4 in│            │             │  current week cycle?        │
 │ current week cycle? │                  ▼             └──────────────┬─────────────┘
 └──────────┬──────────┘        ┌────────────────────────┐             │
            │                   │ Less the 45% of High    │          ┌──────┐
            │        ┌──────┐   │ Aerobic Load in Label 4 in│        │ Yes  │
            │        │  No  │──►│ current week cycle?     │          └──┬───┘
         ┌──────┐    └──┬───┘   └───────────┬────────────┘             │
         │  No  │       │                   │                          ▼
         └──┬───┘    ┌──────┐            ┌──────┐          ┌───────────────────────┐
            │        │ Yes  │            │ Yes  │          │   Workout Label 3     │
            │        └──┬───┘            └──┬───┘          └───────────┬───────────┘
            ▼           │                   ▼                          │
 ┌─────────────────────┐◄┘      ┌────────────────────────┐            │
 │   Workout Label 5   │        │    Workout Label 4     │            │
 └──────────┬──────────┘        └───────────┬────────────┘            │
            │                               │                         │
            │          ┌──────────────────────────────────────┐       │
            └─────────►│   Current weekly training load        │◄──────┘
                       │   compared to weekly training load    │
                       └──────────────────┬───────────────────┘
                                          │
                                          ▼
                       ┌──────────────────────────────────────┐
                       │ Optionally increase training load for │
                       │           next workout                │
                       └──────────────────┬───────────────────┘
                                          │
                                          ▼
                       ┌──────────────────────────────────────┐
                       │   Determine Workout Structure         │
                       └──────────────────────────────────────┘
```

## FIG. 3C

# Workout label path 2-6-7

**FIG. 3D**

| ANAEROBIC FEEDBACK PHRASE LOGIC | | | | | | | |
|---|---|---|---|---|---|---|---|
| | | Anaerobic TE | | | | | |
| | Other conditions | 0-0.9 | 1.0-1.9 | 2.0-2.9 | 3.0-3.9 | 4.0-4.9 | 5.0 |
| | | Only anaerobic phrases 0-4 and 15 are possible when "gym" flag is selected for a workout<br>Other rules related to use of ETE library:<br>Speed inputted to ETE only in running exercises. Power inputted to ETE only in cycling workouts | | | | | |
| Power | No other conditions | #0 | #15 | #1 | #2 | #3 | #4 |
| c1<br>Speed | Peak modified intensity >140% in 5 or more repeats (scale 12-17MET => 150%-130%) AND each repeat lasting 20sec or less | #0 | #5 | #6 | #12 | #13 | #4 |
| c2<br>Power | Average mod intensity > 115% for a total of 75 sec or more AND in 3 or more repeats AND each repeat lasting 10 sec or more (scale 12-17MET => 120%-110%) | #0 | #15 | #7 | #8 | #3 | #4 |
| c3<br>Power | Average mod intensity > 95% for a total of 150 sec or more AND in 7 or more repeats (scale 12-17MET => 100%-95%) AND each repeat lasting 20sec or more AND aerTE < 4.0 | #0 | #15 | #10 | #11 | #14 | #4 |

## FIG. 4

| AEROBIC FEEDBACK PHRASE LOGIC | | | | | | | | | | | |
|---|---|---|---|---|---|---|---|---|---|---|---|
| | | Aerobic TE | | | | | | | | | |
| | Other conditions | 0-0.9 | 1 | 1.5 | 2 | 2.5 | 3 | 3.5 | 4 | 4.5 | 5 |
| Recovery/ Tempo | No other conditions | #18 | #0 | | #1 | | #2 | | #3 | | #4 |
| c1: Recovery/ Tempo | 10min ≤ Duration ≤ 40min AND anTE < 1,0 | #18 | #5 | | #1 | | #2 | | #3 | | #4 |
| c2: Recovery/ Base | Duration > 40min AND Time in HR zone > 95%LTHR for less than 8% of total duration AND C4t2+C5t2 less than 15% of total duration AND anTE <4.0 AND time at 61-92% LTHR > 35min | #18 | #0 | #6 | #7 | | #8 | #9 | #10 | | #11 |
| c3: Recovery/ Tempo | Cumulative time<br>1. @ HR zone 90-96%LTHR equal to or more than 18-24min AND cumulative time equal to or more than 20% of total exercise time<br>2. OR ETEspeed @ 90-96% of AnT speed ≥ 18-24 min AND cumulative time equal to or more than 22% of total exercise time<br>3. OR 30sec_avg_power @ 76-92% of Antpower) ≥ 24-32 min AND "interval not detected " AND cumulative time equal to or more than 25% of total exercise time<br><br>AND anTE < 4.0 | #18 | #0 | | #21 | | #22 | | #23 | | #24 |
| c4: Recovery/ LT | Cumulative time<br>1. @ 94-102% LTHR AND @ modified intensity < 95% VO2max equal to or more than 10-15 min AND cumulative time equal to or more than 15% of total exercise time<br>OR<br>2. @ ETEspeed 94-105% of AnT speed equal to or more than 10-15min AND cumulative time equal to or more than 15% of total exercise time<br>OR<br>3. @ 30sec_avg_power 90-105% of AnT power equal to or more than 13-18 min AND "interval not detected " AND cumulative time equal to or more than 20 % of total exercise time<br><br>AND anTE < 4.0 | #18 | #0 | | #19 | | #12 | | #13 | | #14 |
| c5: Recovery/ VO2max | If duration ≥ 5min AND cumulative time at one of the zones (see below; either based on % LTHR or %ETEspeed or %AnT power) in zone greater than 8 min or more than 80% of total duration<br><br>else if..<br><br>Cumulative time<br>1. @ ≥ 100% LTHR AND modified intensity ≤ 103% equal to or more than 8-12 min<br>OR<br>2. @ ETEspeed ≥ 102% of AnT speed AND modified intensity ≤ 103% equal to or more than 8-12min min AND cumulative time equal to or more than 10% of total exercise time<br>OR<br>3. @ 30sec_avg_power higher than 102% of AnT power AND smaller than 127% of AnT power equal to or more than 8-12 min AND "interval not detected "<br><br>AND cumulative time equal to or more than 15% of total exercise time | #18 | #0 | | #20 | | #15 | | #16 | | #17 |

## FIG. 5

FIG. 6

ANAEROBIC
#7 Speed
#6 Anaerobic Capacity

AEROBIC HIGH INTENSITY
#5 VO2max
#4 Lactate Threshold
#3 Tempo

AEROBIC LOW INTENSITY
#2 Aerobic base
#1 Recovery

| Feedback phrase number | Short TE Feedback | AEROBIC Workout label |
|---|---|---|
| #0 | Easy - aerobic | #1 Recovery |
| #1 | Maintaining - aerobic | #2 Aerobic base |
| #2 | Improving - aerobic | #3 Tempo |
| #3 | Highly improving - aerobic | #3 Tempo |
| #4 | Overreaching - aerobic | #3 Tempo |
| #5 | Easy - recovery | #1 Recovery |
| #6 | Easy - aerobic base | #2 Aerobic base |
| #7 | Maintaining - aerobic base | #2 Aerobic base |
| #8 | Improving - aerobic base | #2 Aerobic base |
| #9 | Improving - aerobic endurance | #2 Aerobic base |
| #10 | Highly Improving - aerobic endurance | #2 Aerobic base |
| #11 | Overreaching - aerobic endurance | #2 Aerobic base |
| #12 | Improving - lactate threshold | #4 Lactate Threshold |
| #13 | Highly improving - lactate threshold | #4 Lactate Threshold |
| #14 | Overreaching - lactate threshold | #4 Lactate Threshold |
| #15 | Improving - VO2max | #5 VO2Max |
| #16 | Highly Improving - VO2max | #5 VO2Max |
| #17 | Overreaching - VO2max | #5 VO2Max |
| #18 | No aerobic effect | NaN |
| #19 | Maintaining - lactate threshold | #4 Lactate Threshold |
| #20 | Maintaining - VO2max | #5 VO2Max |
| #21 | Maintaining - tempo | #3 Tempo |
| #22 | Improving - tempo | #3 Tempo |
| #23 | Highly Improving - tempo | #3 Tempo |
| #24 | Overreaching - tempo | #3 Tempo |

| Feedback phrase number | Short TE Feedback | ANAEROBIC Workout label |
|---|---|---|
| #0 | No anaerobic effect | NaN |
| #1 | Maintaining Anaerobic Fitness | #6 Anaerobic capacity |
| #2 | Improving Anaerobic Fitness | #6 Anaerobic capacity |
| #3 | Highly improving Anaerobic Fitness | #6 Anaerobic capacity |
| #4 | Overreaching | #6 Anaerobic capacity |
| #5 | Easy – Speed | #7 Speed |
| #6 | Maintaining – Speed | #7 Speed |
| #7 | Maintaining – Anaerobic power | #6 Anaerobic capacity |
| #8 | Improving - Anaerobic power & capacity | #6 Anaerobic capacity |
| #10 | Maintaining – Anaerobic base & economy | #6 Anaerobic capacity |
| #11 | Improving – Anaerobic base & economy | #6 Anaerobic capacity |
| #12 | Improving – Anaerobic capacity & speed | #7 Speed |
| #13 | Highly Improving - Anaerobic capacity & speed | #7 Speed |
| #14 | Highly improving – Anaerobic base & economy | #6 Anaerobic capacity |
| #15 | Minor anaerobic effect | #6 Anaerobic capacity |
| #16 | Overreaching - Speed | #7 Speed |

50

EP 3 923 215 A1

| ANAEROBIC FEEDBACK PHRASES | | | |
|---|---|---|---|
| Feedback phrase number | Workout label | Short TE Feedback | Long TE Feedback |
| #0 | NaN | No anaerobic effect | No anaerobic effect due to too little or lacking supramaximal work |
| #1 | #6 Anaerobic capacity | Maintaining Anaerobic Fitness | This exercise maintained anaerobic performance brought on by moderate amount of supramaximal exertion. |
| #2 | #6 Anaerobic capacity | Improving Anaerobic Fitness | This exercise improved anaerobic performance brought on by high amount of supramaximal exertion. |
| #3 | #6 Anaerobic capacity | Highly Improving Anaerobic Fitness | This exercise was very efficient for improving anaerobic power production and anaerobic capacity brought on by extensive amount of supramaximal exertion. |
| #4 | #6 Anaerobic capacity | Overreaching | This was a very hard anaerobic training session. This kind of exercise should be performed only occasionally. Special attention on recovery. |
| #5 | #7 Speed | Easy - Speed | This exercise had a good effect on speed and fast force production capacity brought on by high speed/power of repeats. |
| #6 | #7 Speed | Maintaining - Speed | This exercise had a significant effect on speed and fast force production capacity brought on by high speed/power repeats. |
| #7 | #6 Anaerobic capacity | Maintaining - Anaerobic power | This exercise maintained anaerobic power production capability brought on by high intensity repeats |
| #8 | #6 Anaerobic capacity | Improving - Anaerobic power & capacity | This exercise improved anaerobic power production and anaerobic capacity brought on by high intensity repeats |
| #9 * | #7 Speed | Very easy - Speed | This exercise had a minor effect on speed and fast force production capacity brought on by high speed/power repeats. |
| #10 | #6 Anaerobic capacity | Maintaining - Anaerobic base & economy | This exercise maintained anaerobic base and economy of movement brought on by moderate duration of anaerobic work periods. |
| #11 | #6 Anaerobic capacity | Improving - Anaerobic base & economy | This exercise improved anaerobic base and economy of movement brought on by long duration of anaerobic work periods. |
| #12 | #7 Speed | Improving - Anaerobic capacity & Speed | This exercise improved anaerobic capacity and speed brought on by high speed/power of repeats. |
| #13 | #7 Speed | Highly Improving - Anaerobic capacity & Speed | This exercise sharply improved anaerobic capacity and speed brought on by high speed/power of repeats. |
| #14 | #6 Anaerobic capacity | Highly Improving - Anaerobic base & economy | This exercise sharply improved anaerobic base and economy of movement brought on by extensive duration of anaerobic work periods. |
| #15 | #6 Anaerobic capacity | Minor anaerobic effect | This exercise had a minor anaerobic benefit brought on by detected few anaerobic work periods. |
| #16 | #7 Speed | Overreaching - Speed | This activity was very demanding. While it can significantly improve your anaerobic capacity, power and speed, it can become harmful without enough recovery time and should be done sparingly. |

## FIG. 7

| | | AEROBIC FEEDBACK PHRASES | |
|---|---|---|---|
| Feedback phrase number | Workout label | Short TE Feedback | Long TE Feedback |
| #0 | #1Recovery | Easy - aerobic | This was an easy aerobic exercise that had only a minor effect on your cardiorespiratory fitness. |
| #1 | #2Aerobic base | Maintaining - aerobic | This aerobic exercise session maintained your cardiorespiratory fitness. |
| #2 | #3Tempo | Improving - aerobic | This aerobic exercise session was hard enough for purposes of improving cardiorespiratory fitness. |
| #3 | #3Tempo | Highly improving - aerobic | This aerobic exercise session was very hard and is expected to cause sharp increase in cardiorespiratory fitness. |
| #4 | #3Tempo | Overreaching- aerobic | This aerobic exercise was very demanding for your body and fitness benefits occur only after prolonged recovery. These type of exercises should only be performed occasionally. |
| #5 | #1Recovery | Easy - recovery | This was an easy aerobic exercise. These type of exercises facilitate your recovery after harder training and helps in relieving mental stress. |
| #6 | #2Aerobic base | Easy - Aerobic base | This was an easy aerobic exercise. It had minor benefits for your aerobic base. |
| #7 | #2Aerobic base | Maintaining - aerobic base | Intensity and duration of this workout were high enough to maintain your aerobic base. |
| #8 | #2Aerobic base | Improving - aerobic base | This was a good exercise for improving aerobic base. These type of exercises together with easier aerobic base exercises form the basis of good training plan. |
| #9 | #2Aerobic base | Improving - aerobic endurance | This exercise improved your aerobic base as well as capability to endure exertion for prolonged periods. |
| #10 | #2Aerobic base | Highly improving - aerobic endurance | This exercise was very efficient in improving your aerobic base and capability to endure prolonged exertion. |
| #11 | #2Aerobic base | Overreaching - aerobic endurance | This was a very demanding exercise requiring long recovery. This exercise mainly improved your capability to endure prolonged exertion. |
| #12 | #4Lactate Threshold | Improving - lactate threshold | This was a good exercise for improving your lactate threshold! |

## FIG. 8A

| AEROBIC FEEDBACK PHRASES | | | |
|---|---|---|---|
| Feedback phrase number | Workout label | Short TE Feedback | Long TE Feedback |
| #13 | #4Lactate Threshold | Highly improving - lactate threshold | This exercise was very efficient in improving your lactate threshold! |
| #14 | #4Lactate Threshold | Overreaching - lactate threshold | This was a very demanding exercise requiring long recovery. Main physiological benefit in lactate threshold improvement. |
| #15 | #5VO2max | Improving - VO2max | This was a good exercise for improving your VO2max! |
| #16 | #5VO2max | Highly improving - VO2max | This exercise was very efficient in improving your VO2max! |
| #17 | #5VO2max | Overreaching - VO2max | This was a very demanding exercise requiring long recovery. Main physiological benefit in VO2max improvement. |
| #18 | NaN | No aerobic effect | This exercise did not provide any benefit for cardiorespiratory fitness. |
| #19 | #4Lactate Threshold | Maintaining - Lactate threshold | This exercise included enough high intensity effort to maintain your Lactate Threshold. |
| #20 | #5VO2max | Maintaining - VO2max | This exercise included enough high intensity effort to maintain your VO2max. |
| #21 | #2Tempo | Maintaining - Tempo | This exercise included some high intensity effort at [Marathon pace (running) OR Tempo zone (cycling)] . This type of effort level can be used to develop muscular endurance when performed a bit longer or using shorter recovery periods between intervals. |
| #22 | #2Tempo | Improving - Tempo | This exercise included enough high intensity effort at [Marathon pace (running) OR Tempo zone (cycling)] to improve your muscular endurance. |
| #23 | #2Tempo | Highly improving - Tempo | This was a really good exercise for developing muscular endurance. |
| #24 | #2Tempo | Overreaching - Tempo | This was a really demanding exercise and very efficient for developing muscular endurance. |

## FIG. 8B

**FIG. 9**

**FIG. 10**

**FIG. 11**

| | Europäisches Patentamt European Patent Office Office européen des brevets | **EUROPEAN SEARCH REPORT** | Application Number EP 20 39 7508 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/001134 A1 (RAUHALA KARI KRISTIAN [US] ET AL) 2 January 2020 (2020-01-02) * paragraphs [0010] - [0033] * ----- | 1-19 | INV. G06Q10/06 |
| X | US 2018/085630 A1 (CAPELL REBECCA LYNN [US] ET AL) 29 March 2018 (2018-03-29) * paragraphs [0022] - [0035] * ----- | 1-19 | |
| X | US 2016/220866 A1 (FEICHTINGER RICHARD [DE] ET AL) 4 August 2016 (2016-08-04) * paragraphs [0022] - [0030] * ----- | 1-19 | |
| X | US 2020/101351 A1 (PAGE GEORGE [US]) 2 April 2020 (2020-04-02) * paragraphs [0018] - [0021] * ----- | 1-19 | |

TECHNICAL FIELDS SEARCHED (IPC)

G06Q

The present search report has been drawn up for all claims

| Place of search | Date of completion of the search | Examiner |
|---|---|---|
| Munich | 28 September 2020 | Breidenich, Markus |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 39 7508

This annex lists the patent family members relating to the patent documents cited in the above-mentioned European search report.
The members are as contained in the European Patent Office EDP file on
The European Patent Office is in no way liable for these particulars which are merely given for the purpose of information.

28-09-2020

| Patent document cited in search report | | Publication date | Patent family member(s) | | Publication date |
|---|---|---|---|---|---|
| US 2020001134 | A1 | 02-01-2020 | NONE | | |
| US 2018085630 | A1 | 29-03-2018 | CN | 109791800 A | 21-05-2019 |
| | | | EP | 3520068 A1 | 07-08-2019 |
| | | | TW | 201820215 A | 01-06-2018 |
| | | | US | 2018085630 A1 | 29-03-2018 |
| | | | US | 2019269971 A1 | 05-09-2019 |
| | | | WO | 2018063993 A1 | 05-04-2018 |
| US 2016220866 | A1 | 04-08-2016 | US | 2016220866 A1 | 04-08-2016 |
| | | | WO | 2016120725 A1 | 04-08-2016 |
| US 2020101351 | A1 | 02-04-2020 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20190214144 A
- US 2017143262 A1
- US 10580532 B
- US 10123730 B
- US 9517028 B
- US 9693727 B
- US 7805186 B2
- US 8052580 B2
- US 8465397 B2